# EUROPÄISCHE PATENTSCHRIFT

(11) **EP 2 606 128 B1**
(45) Veröffentlichungstag und Bekanntmachung des Hinweises auf die Patenterteilung: **10.09.2014**
(21) Anmeldenummer: 11781731.2
(22) Anmeldetag: 16.08.2011
(51) Int. Cl.: C07K 14/005

(54) **PERMANENTE HUMANE AMNIOZYTEN ZELLINIE ZUR HERSTELLUNG VON INFLUENZAVIREN**
PERMANENT HUMAN AMNIOCYTIC CELL LINE FOR THE PRODUCTION OF INFLUENZA VIRUS
LIGNEE HUMAINE D' AMNIOCYTE POUR LA PRODUCTION DU VIRUS INFLUENZA

(30) Priorität: 13.05.2011 DE 102011050353; 16.08.2010 DE 102010037008
(43) Veröffentlichungstag der Anmeldung: 26.06.2013
(73) Patentinhaber: CEVEC Pharmaceuticals GmbH, 51105 Köln (DE)
(72) Erfinder: SCHIEDNER, Gudrun, 51105 Köln (DE); REICHL, Udo, 39104 Magdeburg (DE)
(74) Vertreter: Drexl, Janna
(86) Internationale Anmeldenummer: PCT/DE2011/075194
(87) Internationale Veröffentlichungsnummer: WO 2012/041311

(56) Entgegenhaltungen:
- EP-A2- 1 948 789
- WO-A2-01/36615
- WO-A2-02/40665
- WO-A2-2006/071563
- WO-A2-2007/048089
- WO-A2-2008/105931
- US-A1- 2005 170 463
- SCHIEDNER GUDRUN ET AL: "Efficient and reproducible generation of high-expressing, stable human cell lines without need for antibiotic selection", BMC BIOTECHNOLOGY, BIOMED CENTRAL LTD. LONDON, GB, Bd. 8, Nr. 1, 12. Februar 2008 (2008-02-12), Seite 13, XP021035682, ISSN: 1472-6750

## Beschreibung

Die vorliegende Erfindung betrifft ein Verfahren zur Herstellung eines Influenzavirus-basierten Impfstoffs mittels permanenten humanen Amniozytenzellen, sowie die Verwendung einer permanenten humanen Amniozytenzelle zur Herstellung eines Influenzavirus-basierten Impfstoffs.

Die Schutzimpfung stellt die wichtigste Maßnahme im Gesundheitswesen dar, um Erkrankungen vorzubeugen, die durch die jährliche Influenza-Epidemie verursacht werden. Die erfolgreiche Verwendung von Impfstoffen ist davon abhängig, dass möglichst schnell ausreichend große Mengen an Impfmaterial z.B. abgetötete Viren aus stabilen und einfach zu handhabenden Quellen zur Verfügung gestellt werden können. Die schnelle Entwicklung von Impfstoffen und ihre ausreichende Verfügbarkeit sind entscheidend in der Bekämpfung von vielen humanen und tierischen Erkrankungen. Aufgrund von Verzögerungen in der Produktion von Impfstoffen und quantitativen Einbußen können Probleme bei der Bekämpfung der Ausbrüche der Krankheit auftreten. Demzufolge, konzentrieren sich die jüngsten Anstrengungen auf die Anzucht von Viren in Zellkultur zur Verwendung als Impfstoffe.

Bisher werden die erhältlichen Influenzaimpfstoffe in bebrüteten Hühnereiern produziert. Diese Hühnereier müssen nachgewiesenermaßen frei von bestimmten viralen und bakteriellen Kontaminationen sein. Diese sogenannten "specific pathogen free" (SPF) Hühnereier sind kommerziell verfügbar. Obwohl sich Hühnereier in der Vermehrung von tierischen und humanen Viren als sehr geeignet erwiesen haben, bringen sie einige Nachteile in der Impfstoffherstellung mit sich. Da für die Herstellung einer Dosis eines konventionellen Impfstoffs jeweils ein Hühnerei nötig ist, besteht zum Beispiel im Falle einer Pandemie, ein hoher Bedarf an Hühnereiern für die Impfstoffherstellung. Angesichts der begrenzten Verfügbarkeit von Hühnereiern ist mit einem Vorlauf von etwa einem Jahr für die Bereitstellung der Hühnereier in ausreichender Menge zu rechnen. Ferner gibt es Influenzasubtypen, die für Hühner hochpathogen sind, so dass es im Fall einer Pandemie zu Versorgungsengpässen mit Hühnereiern kommen könnte. Zudem ist der Produktionsprozess sehr kostenintensiv und zeitaufwendig. Ein weiterer Nachteil der Impfstoffherstellung in Hühnereiern ist, dass diese Impfstoffe meist nicht frei von Hühnereiweiß sind und somit allergische Reaktionen bei einigen Patienten auftreten können. Nicht zuletzt erfordert die mögliche Selektion von Subpopulation, die sich vom natürlich vorkommenden Virus unterscheiden, alternative Wirtszellsysteme.

Im Gegensatz zu Hühnereiern sind Zellen für die Grippeimpfstoffproduktion auf Zellkultur-Basis stets vorrätig. Sie werden tiefgefroren gelagert und können jederzeit bei Bedarf in der benötigten Menge kurzfristig aufgetaut und vermehrt werden. Mit der Impfstoffproduktion kann daher zu jedem gewünschten Zeitpunkt begonnen werden. Im Fall eines unerwartet hohen Bedarfs oder auch wenn unvorhergesehen neue Virusstämme vermehrt zirkulieren, kann kurzfristig ein entsprechender Impfstoff bereitgestellt werden.

Der Produktionsprozess mittels Zellkultur ermöglicht die Produktion von Viren als Impfstoffe in einem geschlossen, standardisierten System unter definierten, kontrollierten Bedingungen. Aufgrund des kontrollierten Produktionsverfahrens kommt der fertige Grippeimpfstoff ohne Zusatz von Antibiotika aus. Da die Herstellung der Zellkultur-Influenzaimpfstoffe vollkommen unabhängig von Hühnereiern erfolgt, weist der so hergestellte Impfstoff kein Hühnereiweiß auf und kann somit keine allergischen Reaktionen bedingt durch Unverträglichkeiten von Hühnereiweiß in Patienten auslösen.

Derzeit werden hauptsächlich die drei folgenden Zelllinien, die humane PER.C6 Zellen, die Madin Darby Canine Kidney (MDCK) Zellen und die Meerkatzen-Nierenzellen (Vero) zur Influenzaimpfstoffherstellung verwendet. Zusätzlich werden derzeit eine Enten-Retina-Zelllinie (AGE1.CR) und embryonale Vogel-Stammzelllinien entwickelt. Die Herstellung von Impfstoffen in Säugerzellen stellt zwar eine Alternative zur Hühnerei-basierten Impfstoffherstellung dar, jedoch benötigen diese Zellen für ihr Wachstum Serum und/oder die Anhaftung an einen festen Träger. Dies erschwert und somit verteuert die Herstellung von Impfstoffen in diesen Zellen, da aus Sicherheitsgründen das Serum vollständig abgetrennt werden muss und das Wachstum an festen Trägern begrenzt ist und damit zu geringeren Ausbeuten führt.

Ein Vorteil der Herstellung von Impfstoffen in Säugerzellen besteht darin, dass es bei der Isolierung und Replikation des Virus in der Zellkultur zu keiner Passagier-abhängigen Selektion eines Phenotyps kommt, der vom klinischen Wildtyp abweicht. Daher sollte das virale Glykoprotein Hämagglutinin, über das die Anheftung an die zu infizierende Zelle und die Integration des Virus in die Zelle erfolgt, in einer nativen Form exprimiert werden und dadurch eine verbesserte Spezifität und Avidität aufweisen und somit eine Zell-vermittelte Immunität in Menschen ermöglichen.

Daher liegt der Erfindung die Aufgabe zu Grunde, verbesserte permanente humane Zelllinien zur Herstellung von Influenzavirus-basierten Impfstoffen bereitzustellen.

Die Aufgabe wird durch den in den Patentansprüchen definierten Gegenstand gelöst.

Die Figuren erläutern die Erfindung.

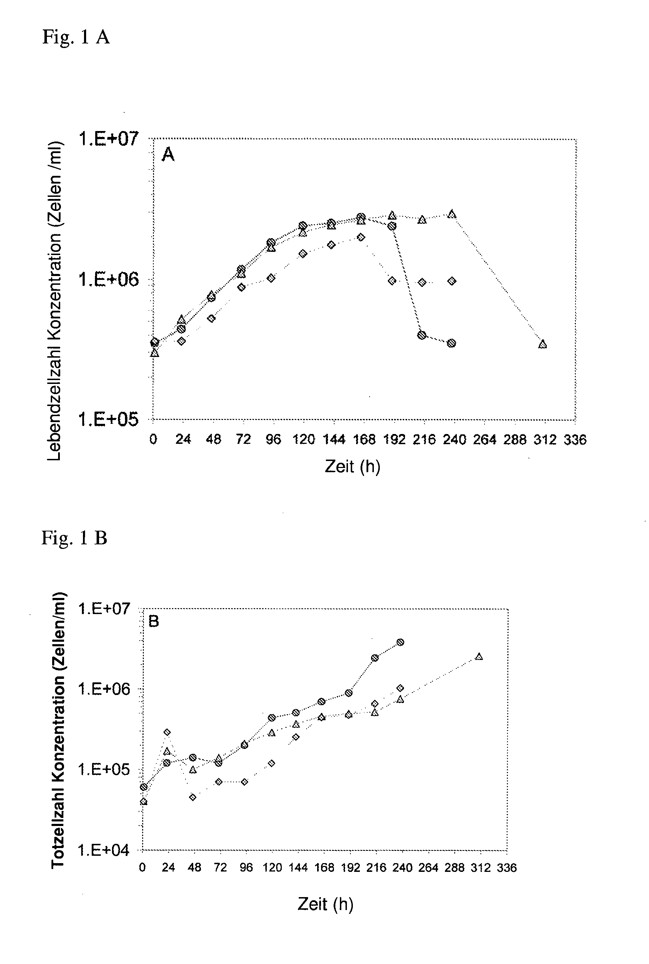

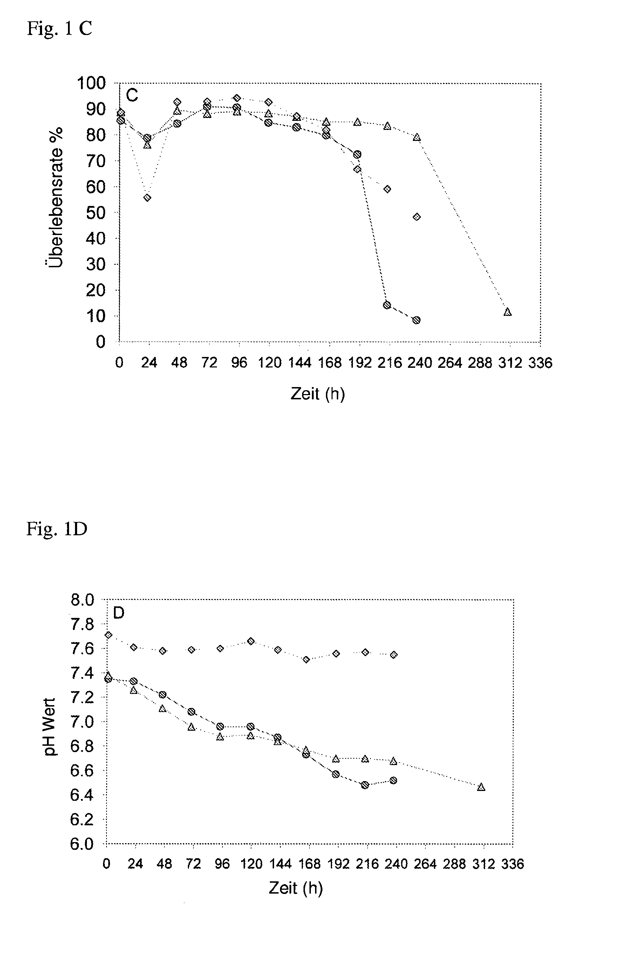

Figur 1 A bis G zeigt schematisch den Verlauf unterschiedlicher Parameter während der Kultivierung der permanenten Amniozytenzelllinie CAP-1D5 in 293SFMII-Medium (●), der permanenten Amniozytenzelllinie CAP-1D5 in PEM-Medium (▲) und der permanenten Hundenierenzelllinie MDCK.SUS2 (Madin Darby Canine Kidney) in SMIF8-Medium (◆) in 100 ml Schüttelkolben. In Fig. 1A ist graphisch der Verlauf der Lebendzellzahl der drei Zelllinien im Vergleich dargestellt, in Fig. 1B der Verlauf der Totzellzahl der Zelllinien und in Fig. 1C der Verlauf der Überlebensrate der Zelllinien. Die Figuren 1 D bis G zeigen schematisch den Verlauf des pH-Werts (D), der Glucose- (helle Symbole) und Laktose-(dunkle Symbole) Konzentration (E), der Glutamin- (Gln) (helle Symbole) und Ammonium-(dunkle Symbole) Konzentration (F) und der Glutaminsäure- (Glu) (helle Symbole) und Pyruvat- (dunkle Symbole) Konzentration (G).

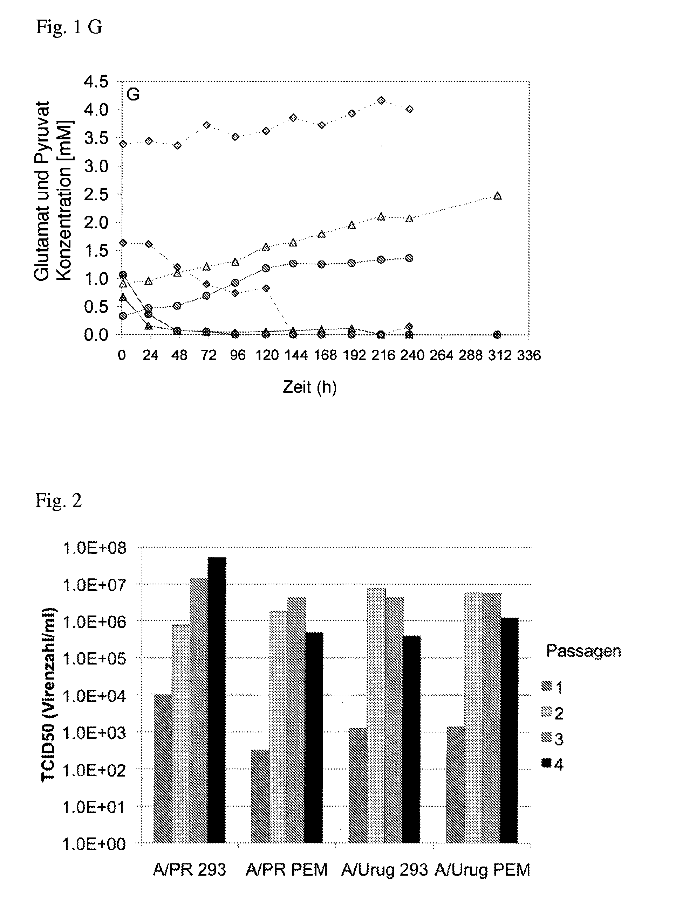

Figur 2 zeigt ein Säulendiagramm, das die Virustiter gemessen als TCID₅₀-Wert über 4 Passagen der Influenza-Stämme A/PR/8/34 (H1N1) und A/Uruguay/716/2007 (H3N2) an CAP-1D5-Zellen in 293SFMII- und PEM-Medium wiedergibt. Abkürzungen: A/PR 293: Influenza-Stamm A/PR/8/34 (H1N1) an CAP-1D5-Zellen in 293SFMII-Medium; A/PR PEM: Influenza-Stamm A/PR/8/34 (H1N1) an CAP-1D5-Zellen in PEM-Medium; A/Urug 293: Influenza-Stamm A/Uruguay/716/2007 (H3N2) an CAP-1D5-Zellen in 293SFMII-Medium; A/Urug PEM: Influenza-Stamm A/Uruguay/716/2007 (H3N2) an CAP-1D5-Zellen in PEM-Medium; TCID₅₀-Wert ist der Virustiter in Virenzahl/ml, der nötig ist, um 50 % der Wirtszellen zu infizieren.

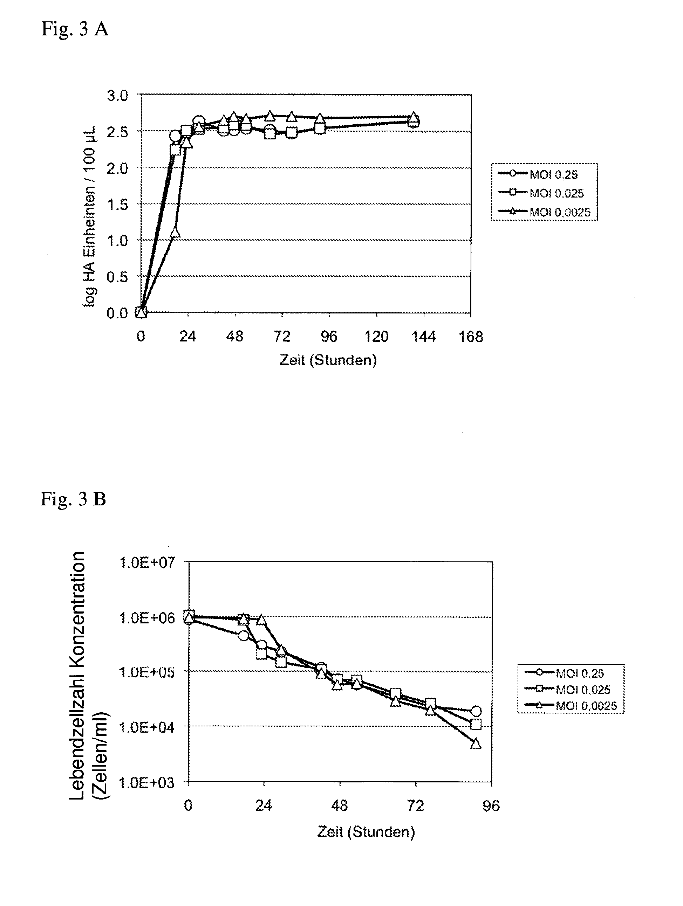

Figur 3 A bis F zeigt schematisch den Verlauf der Menge an Virenpartikeln angegeben als log HA- (Hämagglutinin) Einheiten/100µl und der Lebendzellzahl bei Kultivierung der permanenten Amniozytenzellen CAP-1D5 in 293SFMII- (A, B) und in PEM-Medium (C, D), sowie der permanenten Hundenierenzellen MDCK.SUS2 in SMIF8-Medium (E, F) nach Infektion der Zellen mit dem Influenzavirus-Stamm A/PR/8/34 bei Verwendung unterschiedlicher Virusmengen angegeben als MOI (multiplicity of infection) Werte: MOI: 0,0025 (△); MOI: 0,025 (□); MOI: 0,25 (○). MOI (multiplicity of infection) gibt das Verhältnis aus Anzahl infektiöser Partikel zu Zielzellen wieder.

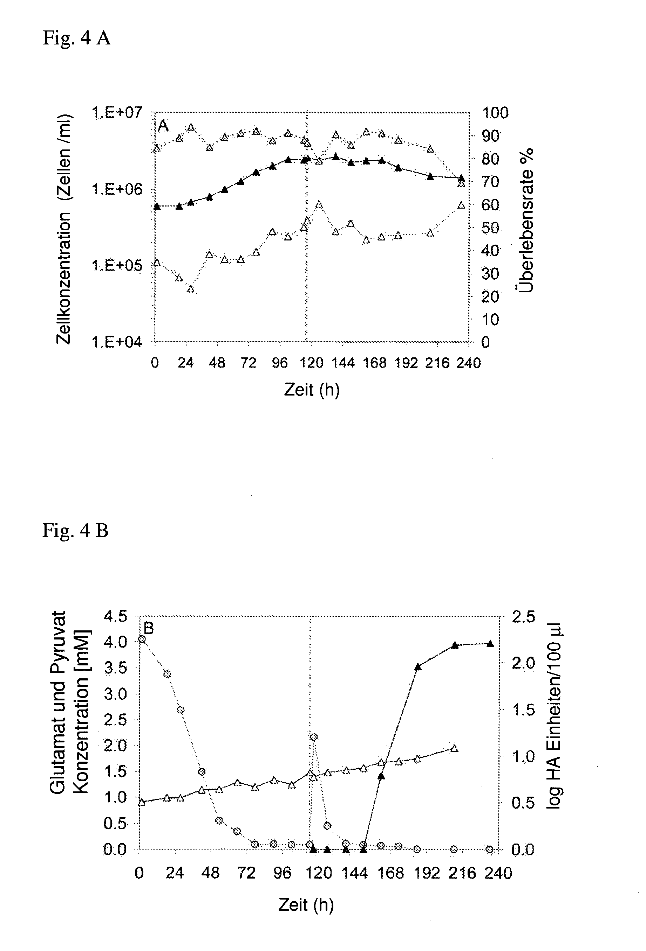

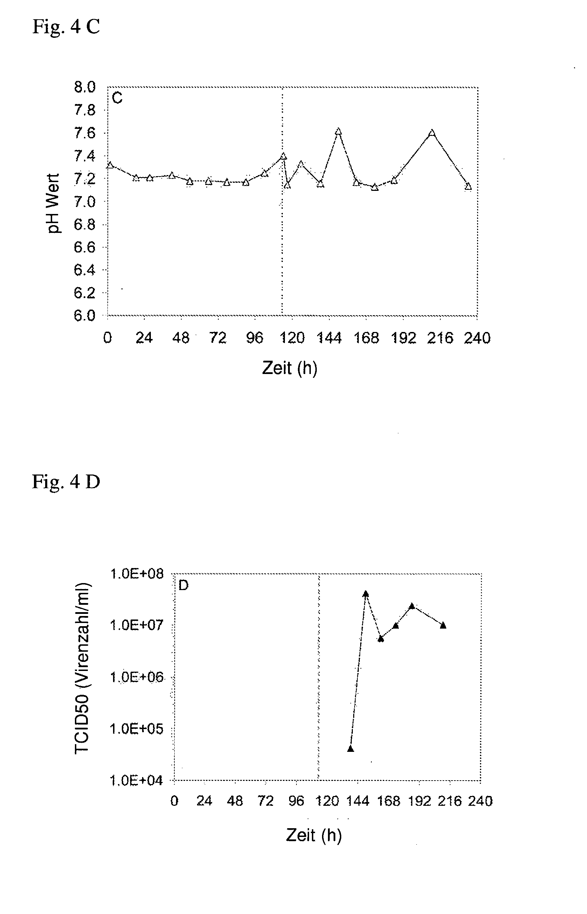

Figur 4 A bis D zeigt schematisch den Verlauf unterschiedlicher Parameter bei der Kultivierung der permanenten Amniozytenzelllinie CAP-1D5 in PEM-Medium im 1 L Bioreaktor, wobei die Infektion nach 114 h mit einer Virusmenge angegeben als MOI von 0,025 mit dem Influenzavirus A/PR/8/34 (adaptiert) stattfindet. In Fig. 4A ist der schematische Verlauf der Lebendzellzahl (▲), Totzellzahl (△) und der Überlebensrate ( 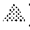 ) der Zellen dargestellt. In Figur 4B ist die Menge an Virenpartikeln angegeben als log HA-(Hämagglutinin) Einheiten/100µl (▲), die Glutamat- (△) und Pyruvat- ( 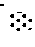 ) Konzentration im Medium schematisch dargestellt. Figur 4 C zeigt schematisch den Verlauf des pH-Wertes (△) und Figur 4 D den Verlauf der Infektiosität (in TCID₅₀ /ml). Der TCID₅₀-Wert gibt den Virustiter in Virenzahl/ml wieder, der nötig ist, um 50 % der Wirtszellen zu infizieren.

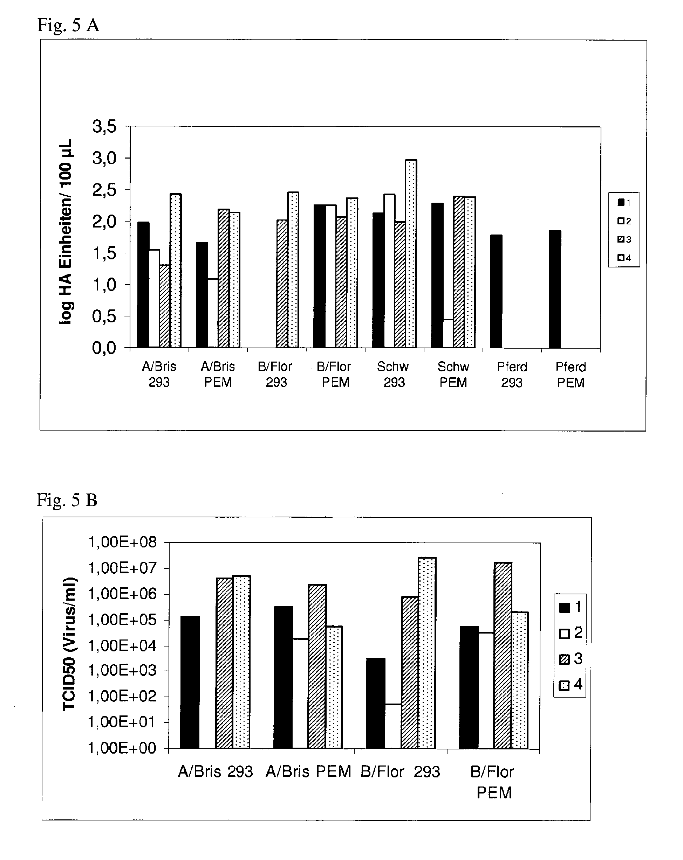

Die Figuren 5 A und B zeigen Säulendiagramme, die die Virustiter gemessen als log HA-Einheiten/100 µl (A) oder TCID₅₀-Wert (B) über 4 Passagen der Influenza-Stämme A/Brisbane/59/2007, B/Florida/4/2006, Schweineinfluenza (A/Swine (H1N2) Bakum/1832/00) und Pferdeinfluenza (A/Equine, A/Newmarket/1/93 (H3N8)) an CAP-1D5-Zellen in 293SFMII- und PEM-Medium wiedergibt. Abkürzungen: A/Bris 293: Influenza-Stamm A/Brisbane/ 59/2007 an CAP-1D5-Zellen in 293SFMII-Medium; A/Bris PEM: Influenza-Stamm A/Brisbane/ 59/2007 an CAP-1D5-Zellen in PEM-Medium; B/Flor 293: Influenza-Stamm B/Florida/4/2006 an CAP-1D5-Zellen in 293SFMII-Medium; B/Flor PEM: Influenza-Stamm B/Florida/4/2006 an CAP-1D5-Zellen in PEM-Medium; Schw 293: Influenza-Stamm A/Swine (H1N2) Bakum/1832/00 an CAP-1D5-Zellen in 293SFMII-Medium; Schw PEM: Influenza-Stamm A/Swine (H1N2) Bakum/1832/00 an CAP-1D5-Zellen in PEM-Medium; Pferd 293: Influenza-Stamm A/Equine, A/Newmarket/1/93 (H3N8) an CAP-1D5-Zellen in 293SFMII-Medium; Pferd PEM: Influenza-Stamm A/Equine, A/Newmarket/1/93 (H3N8) an CAP-1D5-Zellen in PEM-Medium; TCID₅₀-Wert ist der Virustiter in Virenzahl/ml, der nötig ist, um 50 % der Wirtszellen zu infizieren.

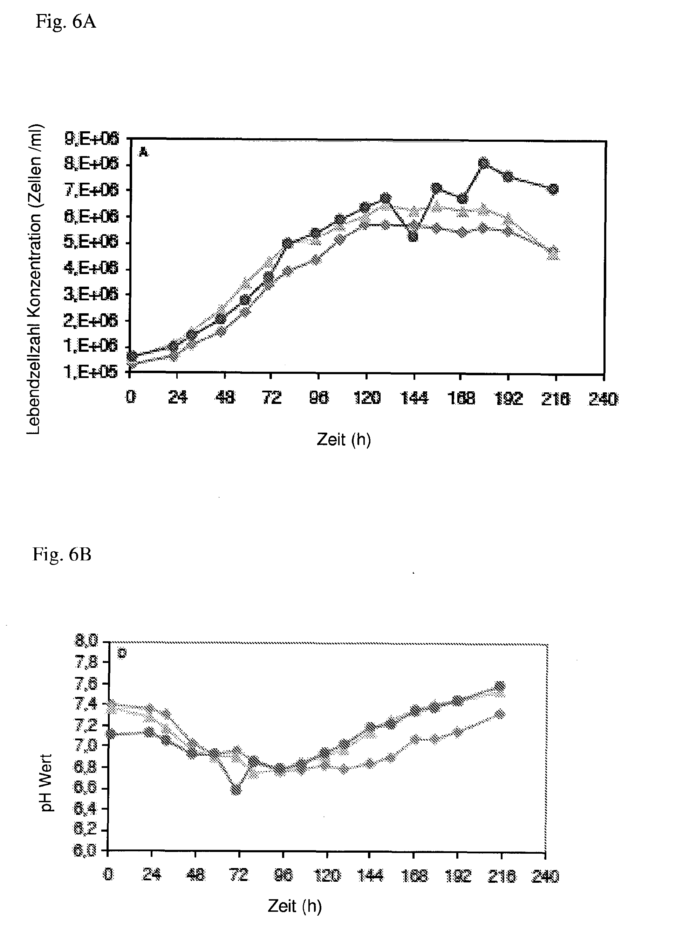

Figur 6 A und B zeigt schematisch den Verlauf der Lebendzellzahl-Konzentration und des pH-Werts bei der Kultivierung der permanenten Amniozytenzelllinie CAP-1D5 in 100 ml PEM-Medium im Schüttelkolben, wobei die Startzellzahl 5 x 10⁵ Zellen/ml beträgt und das Medium zusätzlich 4 mM Pyruvat enthält (▲) oder die Startzellzahl 8 x 10⁵ Zellen/ml beträgt und das Medium zusätzlich 4 mM Pyruvat enthält (▲) oder die Startzellzahl 8 x 10⁵ Zellen/ml beträgt und das Medium zusätzlich 10 mM Pyruvat plus weitere Aminosäuren enthält (●).

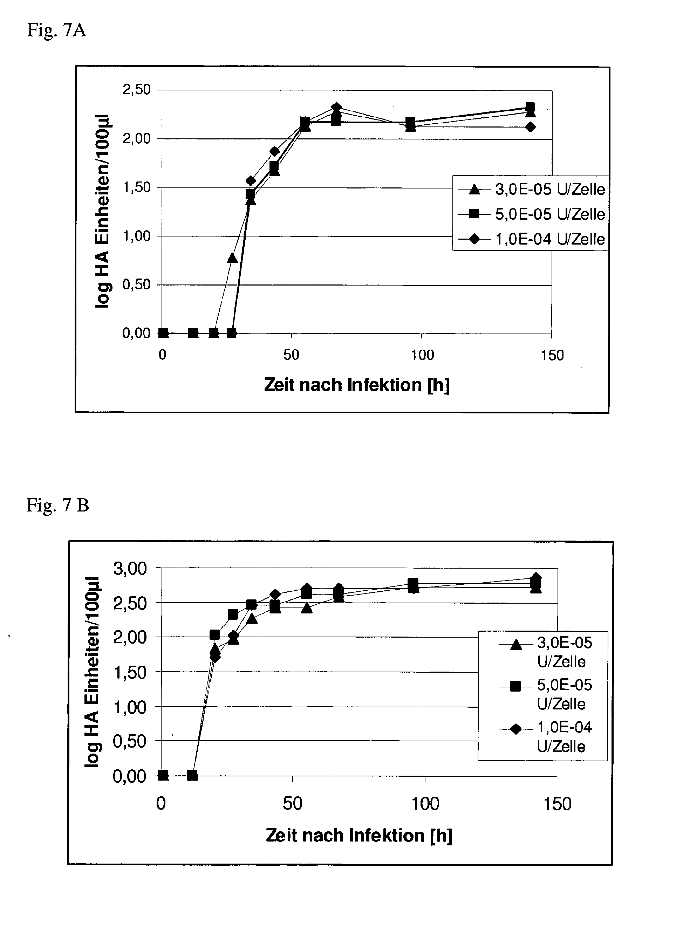

Figur 7 A bis C zeigt schematisch den Verlauf der Virustiter gemessen in log HA-Einheiten/100 µl Kultur, wobei die CAP-1D5-Zellen mit dem adaptierten Influenzastamm A/PR/8/34 infiziert wurden. Vor der Infektion wurde entweder kein Mediumwechsel (A), eine 1:2-Verdünnung mit PEM-Medium (B) oder ein kompletter Mediumwechsel durchgeführt.

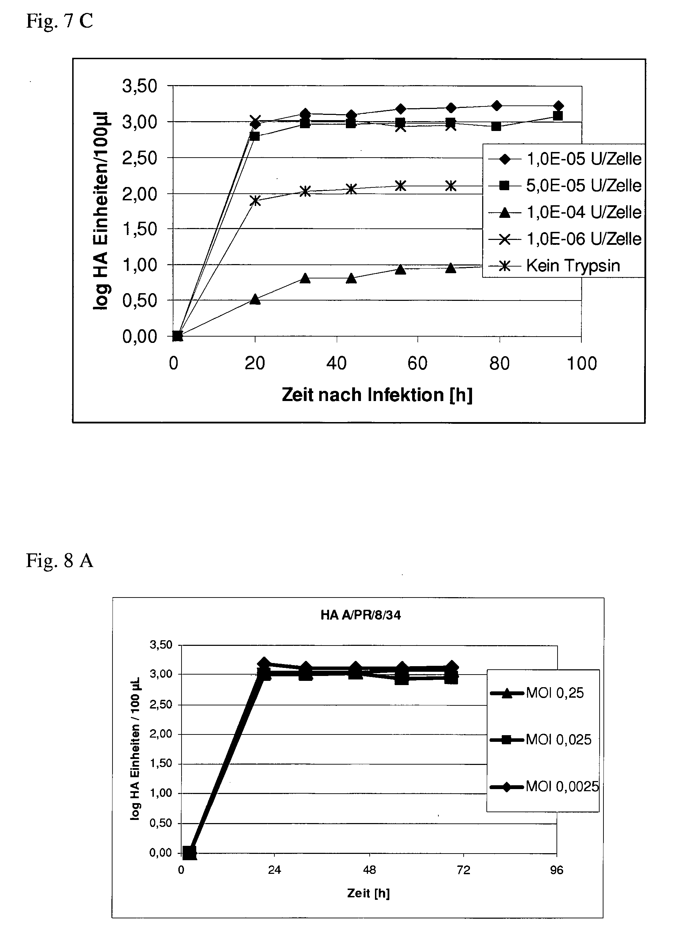

Figur 7 A zeigt den schematischen Verlauf des Virustiters von CAP-1D5-Zellkulturen ohne Mediumwechsel, wobei unterschiedliche Trypsinkonzentrationen von 1 x 10⁻⁴ U/Zelle (◆), 3 x 10⁻⁵ U/Zelle (▲) und 5 x 10⁻⁵ U/Zelle (■) bei der Infektion verwendet wurden. Figur 7 B zeigt den schematischen Verlauf des Virustiters von CAP-1D5-Zellkulturen mit einer 1:2-Verdünnung mit PEM-Medium, wobei unterschiedliche Trypsinkonzentrationen von 1 x 10⁻⁴ U/Zelle (◆), 3 x 10⁻⁵ U/Zelle (▲) und 5 x 10⁻⁵ U/Zelle (■) bei der Infektion verwendet wurden. Figur 7 C zeigt den schematischen Verlauf des Virustiters von CAP-1D5-Zellkulturen mit komplettem Mediumwechsel, wobei entweder kein Trypsin ( 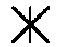 ) oder unterschiedliche Trypsinkonzentrationen von 1 x 10⁻⁴ U/Zelle (▲), 1 x 10⁻⁵ U/Zelle (◆), 5 x 10⁻⁵ U/Zelle (■) und 1 x 10⁻⁶ U/Zelle (x) bei der Infektion verwendet wurden.
Figur 8 A bis F zeigt schematisch den Verlauf der Virustiter in CAP-1D5-Zellkulturen, die mit den Influenzaviren A/PR/8/34, A/Brisbane/59/2007 oder B/Florida/4/2006 infiziert wurden, wobei vor Infektion ein Mediumwechsel erfolgte (A bis C) oder nicht (D bis F). Die Infektion mit dem Influenzastamm A/PR/8/34 und B/Florida/4/2006 erfolgte jeweils mit Virusmengen angegeben als MOI-Wert von 0,25, 0,025 und 0,0025. Die Infektion mit dem Influenzastamm A/Brisbane/59/2007 erfolgte jeweils bei den MOI-Werten von 0,1, 0,025 und 0,0025. MOI (multiplicity of infection) gibt das Verhältnis aus Anzahl infektiöser Partikel zu Zielzellen wieder.

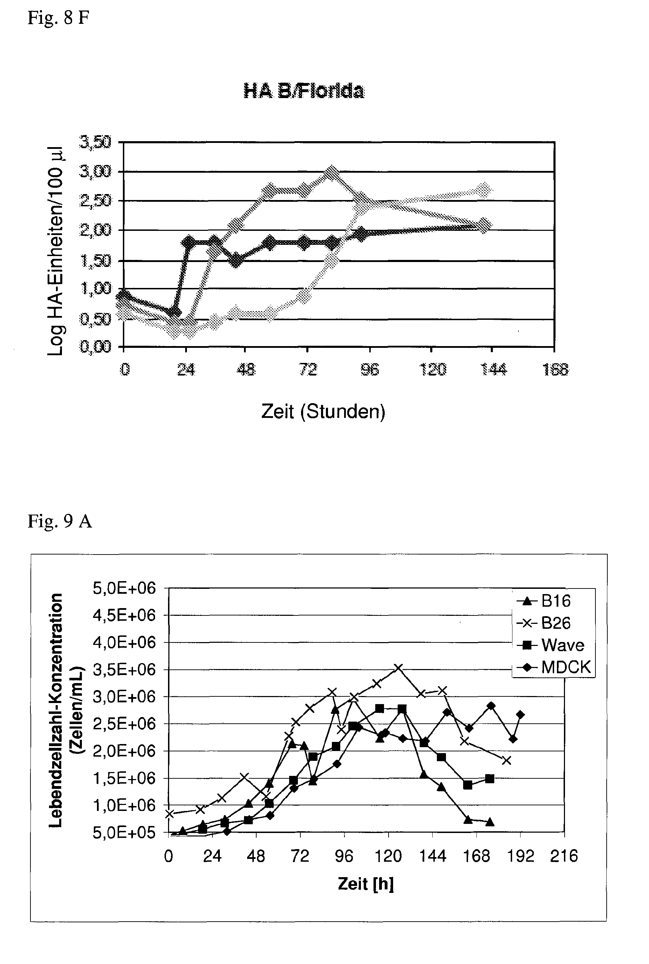

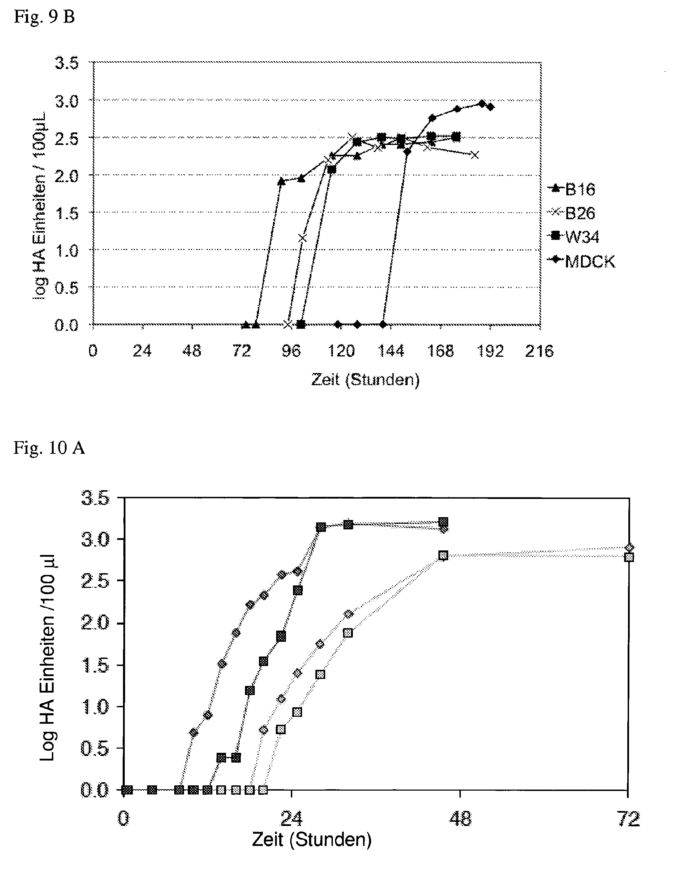

Figur 9 A und B zeigt schematisch den Verlauf der Lebendzellzahl-Konzentration und des Virustiters von CAP-1D5-Zellkulturen (B16, B26 und Wave) und einer Hundenierenzellen-MDCK.SUS2 Kultur (MDCK), die mit adaptiertem Influenzavirus A/PR/8/34 infiziert wurden und im 1 L-Maßstab in STR- (Sartorius) (B16, B26 und MDCK) oder Wave-Bioreaktoren (Wave Biotech AG) (Wave) kultiviert wurden. Vor der Infektion fand bei den Kulturen B26 und Wave ein Mediumwechsel statt.
Figur 10 A bis C zeigt schematisch den Verlauf des Virustiters gemessen in log HA-Einheiten/100 µl, der Lebendzellzahl und des pH-Werts von CAP-1D5-Zellkulturen, die mit adaptiertem Influenzavirus A/PR/8/34 infiziert wurden und in 100 ml PEM-Medium im Schüttelkolben kultiviert wurden. Vor der Infektion fand entweder ein 1:1-Mediumwechsel (helle Symbole) mit 293SFMII-Medium (□) oder PEM-Medium (◊) statt oder ein kompletter Mediumwechsel (dunkle Symbole) mit 293SFMII-Medium (■) oder PEM-Medium (◆).

Unter dem Begriff "Influenzavirus" wie hier verwendet, versteht man Mitglieder der Orthomyxoviren, die Menschen und Tiere infizieren können. Man unterscheidet hierbei Influenzaviren der Typen A, B und C. Influenza-A- und -B-Viren sind in einem Genus zusammengefasst. Die Influenza-C-Viren unterscheiden sich aufgrund ihrer sieben Genomsegmente, bei Influenza-A- und -B-Viren sind es acht Genomsegmente. Zudem kodieren die Influenza-A- und -B-Viren für je ein Hämagglutinin (HA) und eine Neuraminidase (NA), hingegen kodieren die Influenza-C-Viren für ein Oberflächenprotein, das beide Eigenschaften vereinigt, dem Hämagglutinin-Esterase-Fusionsprotein (HEF). Die Influenza-A-Viren werden weiter unterteilt in Untertypen basierend auf der Sequenz von Hämagglutinin- (H1-H15) und Neuraminidase- (N1-N9) Molekülen.

Unter dem Begriff "Influenzavirus-Protein" wie hier verwendet, versteht man Proteine oder Derivate des Influenzavirus. Ein Derivat des Influenzavirus ist typischerweise ein Protein oder ein Teil davon des Influenzavirus, welches zu Immunisierungs-Zwecken verwendet werden kann. Proteine oder Derivate des Influenzavirus umfassen Proteine der Virushülle oder Teile davon. Insbesondere umfassen Influenzavirus-Proteine Influenza-A-Proteine, Influenza-B-Proteine oder Influenza-C-Proteine, z.B. Hämagglutinin (HA), Neuraminidase (NA), Nukleoprotein (NP), die Matrixproteine (M1) und (M2), die Polymeraseproteine (B1), (PB2) und (PA) und die Nichtstrukturproteine (NS1) und (NS2) und Teile davon. Teile der Influenzavirus-Proteine umfassen ein oder mehrere Epitope der Influenza-A-Proteine, Influenza-B-Proteine oder Influenza-C-Proteine. Die Epitope können CD4+ T-Zell-Epitope, die Peptide darstellen, welche ein Bindemotif der Klasse MHC-Klasse-II enthalten und an der Oberfläche Antigen-präsentierender Zellen durch Moleküle der MHC-Klasse-II repräsentiert werden, sein oder CD8+ T-Zell-Epitope, die Peptide darstellen, welche ein Bindemotif der Klasse MHC-Klasse-I enthalten und an der Oberfläche Antigen-präsentierender Zellen durch Moleküle der MHC-Klasse-I repräsentiert werden. Beispielsweise erlauben algorithmische Modelle, MHC-Bindetests, *in silico* Antigen-Identifizierungsverfahren und Röntgen-Kristallographie-Verfahren erlauben die Identifizierung von Antigenen, die verschiedene MHC-Moleküle binden können.

Unter dem Begriff "Impfstoff" wie hier verwendet versteht man ein biologisch oder gentechnisch hergestelltes Antigen, umfassend Proteine, Protein-Untereinheiten, Peptide, Kohlenhydrate, Lipide, Nukleinsäuren, abgetötete oder abgeschwächte Viren, wobei es sich hier um ganze Virenpartikel oder Teile von Virenpartikel handeln kann oder Kombinationen hiervon. Das Antigen kann mindestens ein Epitop z.B. ein T-Zell- und/oder B-Zell-Epitop präsentieren. Dieses Antigen wird von immunologischen Rezeptoren, wie dem T-Zell-Rezeptor oder B-Zell-Rezeptor erkannt. Der Impfstoff dient nach Applikation zur spezifischen Aktivierung des Immunsystems hinsichtlich eines bestimmten Virus. Hierbei wird die Reaktion des Immunsystems ausgenutzt, bei Vorhandensein von Viren bzw. deren spezifischen Antigenen eine Immunantwort hervorzurufen. Diese führt zur Ausbildung von Antikörpern und spezialisierten T-Helferzellen, die einen lange anhaltenden Schutz vor der jeweiligen Krankheit bieten können, der je nach Virus zwischen einigen Jahren und lebenslang anhalten kann. Impfstoffe umfassen Lebend- oder Totimpfstoffe. Der Lebendimpfstoff enthält zum Beispiel abgeschwächte, noch vermehrungsfähige Viren, welche die Krankheit nicht auslösen können. Bei einem Totimpfstoff wurden diese Viren dagegen abgetötet oder er enthält nur noch Bruchstücke des Virus (Antigene). Die Inaktivierung (Abtötung) der Viren erfolgt beispielsweise durch chemische Substanzen, z. B. Formaldehyd, beta-Propiolacton und Psoralen. Die Virushülle bleibt dabei erhalten. Es gibt auch Toxoidimpfstoffe, die nur den biologisch inaktiven Bestandteil (Toxoid) des Toxins eines Virus enthalten (z.B. das Tetanus-Toxoid), die ebenfalls zu den Totimpfstoffen gezählt werden. Insbesondere kann es sich bei dem Totimpfstoff um einen Spaltimpfstoff handeln, der aus Bruchstücken der Virushüllproteine besteht. Die Zerstörung oder Spaltung der Virushülle kann beispielsweise mit Detergentien oder starken organischen Lösungsmitteln erfolgen. Die Viren können auch zusätzlich mit chemischen Stoffen inaktiviert bzw. abgetötet werden. Ferner zählen zu den Totimpfstoffen die Untereinheitimpfstoffe, die aus spezifischen Komponenten des Virus, beispielsweise Hämagglutinin- und Neuraminidase-Proteine bestehen.

Unter dem Begriff "Influenzavirus-basierter Impfstoff" wie hier verwendet, versteht man sämtliche Proteine, Peptide oder Teile davon, sowie Nukleinsäuren kodierend für diese Proteine, Peptide oder Teile davon des Influenzavirus, sowie Influenzaviruspartikel selbst, rekombinante Influenzavirus-Proteine, einschließlich Influenza-Hüllproteine, subvirale Partikel, Virus-ähnliche Partikel (VLP), VLP-Komplexe, und/oder Teile davon, die zu Immunisierungszwecken gegen Influenza verwendet werden können.

Unter dem Begriff "Adjuvant" wie hier verwendet versteht man Substanzen, die die Immunogenität eines Antigens modulieren können. Adjuvantien sind zum Beispiel Mineralsalze, Squalen-Gemische, Muramyl-Peptide, Saponin-Derivate, Mycobakterien-Zellwand-Präparationen, bestimmte Emulsionen, Monophosphoryl-Lipid-A, Mykolsäure-Derivate, nichtionische Block-Copolymer-Tenside, Quil A, Untereinheit des Cholera-Toxins B, Polyphosphazene und dessen Derivate, immunstimulierende Komplexe, Cytokin-Adjuvantien, MF59-Adjuvans, Lipid-Adjuvantien, mukosale Adjuvantien, bestimmte Bakterien-Exotoxine, bestimmte Oligonukleotide und PLG.

Unter dem Begriff "Amniozyten" wie hier verwendet versteht man alle Zellen, die im Fruchtwasser vorhanden sind und durch Fruchtwasserpunktion gewonnen werden können. Sie stammen entweder vom Amnion oder von fetalem Gewebe, das im Kontakt mit der Amnionflüssigkeit ist. Es wurden drei Hauptklassen von Amniozyten beschrieben, die auf Grund morphologischer Kriterien unterschieden werden: Fibroblastenartige Zellen (F-Zellen), epitheloide Zellen (E-Zellen) und Amnionflüssigkeitszellen (Amniotic Fluid Cells, AF-Zellen) (Hohn et al., Pediat. Res. 8:746-754, 1974). AF-Zellen sind der vorherrschende Zelltyp.

Unter dem Begriff "permanente Zelllinien" wie hier verwendet versteht man Zellen, die derart genetisch verändert sind, dass sie unter geeigneten Kulturbedingungen permanent in Zellkultur weiter wachsen können. Solche Zellen werden auch immortalisierte Zellen genannt.

Unter dem Begriff "primäre Zellen" wie hier verwendet versteht man Zellen, die durch direkte Entnahme aus einem Organismus oder einem Gewebe erhalten und in Kultur genommen wurden. Primäre Zellen besitzen nur eine sehr begrenzte Lebensdauer.

Unter dem Begriff "Transfektion" wie hier verwendet wird jedes Verfahren verstanden, das sich zum Einbringen der genannten Nukleinsäure(n) in die Zellen eignet. Als Beispiele sind die klassische Kalziumphosphat-Methode, Elektroporation, liposomale Systeme jeglicher Art und Kombinationen dieser Verfahren zu nennen.

Unter dem Begriff "CAP" wie hier verwendet, versteht man permanente humane Amniozytenzelllinien, die durch Immortalisierung von primären humanen Amniozyten mit adenoviralen Genfunktionen E1A und E1B generiert worden sind.

Unter dem Begriff "CAP-T" wie hier verwendet, versteht man CAP-Zellen, die zusätzlich mit einem Nukleinsäuremolekül enthaltend die Sequenz des SV40 großen T-Antigens stabil transfiziert wurden.

Ein Gegenstand der vorliegenden Erfindung betrifft ein Verfahren zur Herstellung eines Influenzavirus-basierten Impfstoffs gemäß Anspruch 1 umfassend die folgenden Schritte:
(i) Infizieren einer permanenten humanen Amniozytenzelle mit einem Influenzavirus
(ii) Züchten der permanenten humanen Amniozytenzelle
(iii) Expression des Influenzavirus und
(iv) Isolieren des Influenzavirus aus dem Medium, wobei die permanente humane Amniozyteuzelle die adenoiralen Genprodukte EIA und EIB exprimiest. In dem erfindungsgemäßen Verfahren werden permanente humane Zellen unter Bedingungen kultiviert (z.B. Temperatur, Medium, pH-Wert), die für das Wachstum der Zellen geeignet sind. So sind die Bedingungen hinsichtlich der Temperatur, dem Medium, dem pH-Wert und anderen Wachstumsparametern dem Fachmann bekannt, oder können mit den üblichen Verfahren ermittelt werden. Hat die Kultur eine gewünschte Wachstumsdichte erreicht, werden die Influenzaviren zur Infektion der Zellen hinzugegeben. Die Viren benötigen mehrere Tage zur Vermehrung innerhalb der Zellen. Während dieses Vervielfältigungsvorganges stirbt ein großer Teil der Zellen ab und die Viren werden in das Medium freigesetzt. Die virushaltige Lösung wird von den Zellresten durch z.B. Zentrifugation abgetrennt. Das Virus kann dann von der Medienlösung mittels z.B. einer Chromatographie-Säule getrennt werden und das Volumen reduziert werden. Die Viren können anschließend durch z.B. einen chemischen Prozess inaktiviert werden. Danach kann sich eine Virusspaltung anschließen. Nach weiteren Reinigungs- und Konzentrierungsschritten erhält man das Antigenkonzentrat eines Virusstammes.

In einer bevorzugten Ausführungsform werden zur Infektion der permanenten humanen Zellen die Influenzavirusstämme A/PR/8/34, A/Uruguay/716/2007, A/Brisbane/59/2007, B/Florida/4/2006, Schweineinfluenza (A/Swine (H1N2) Bakum/1832/00) oder Pferdeinfluenza (A/Equine, A/Newmarket/1/93 (H3N8)) verwendet.

In einer weiteren bevorzugten Ausführungsform werden die zur Infektion der permanenten humanen Zellen verwendeten Influenzaviren zuvor an die Zellen adaptiert, bevorzugt handelt es sich hierbei um die oben gelisteten Influenzaviren. Bevorzugt erfolgt eine solche Adaption über 4 Passagen. Bevorzugt erfolgt die Adaption der Influenzaviren in 293SFMII-Medium oder PEM-Medium.

Ein weiteres Verfahren zur Herstellung eines Influenzavirus-basierten Impfstoffs umfassend die folgenden Schritte:
(i) In Kontaktbringen eines Nukleinsäuremoleküls kodierend für ein Influenzavirus-Protein mit einer permanenten humanen Zelle,
(ii) Züchten der permanenten humanen Zelle,
(iii) Ermöglichen der Replikation des Nukleinsäuremoleküls kodierend für ein Influenza-Protein und/oder Expression des Influenza-Proteins und
(iv) Isolieren des Nukleinsäuremoleküls kodierend für ein Influenza-Protein und/oder des Influenzavirus-Proteins aus dem Medium.

In einer bevorzugten Ausführungsform handelt es sich bei den in den erfindungsgemäßen Verfahren verwendeten permanenten humanen Zellen um permanente humane Amniozytenzellen.

In einer bevorzugten Ausführungsform werden die permanenten humanen Zellen in Schüttelkolben oder Bioreaktoren, bevorzugt STR- oder Wave-Bioreaktoren kultiviert. Die permanenten humanen Zellen können in unterschiedlichen Medien kultiviert werden, bevorzugt in 293SFMII- oder PEM-Medium. Ferner kann dem Medium Pyruvat, Glutamin, Glucose und weitere Aminosäuren zugesetzt werden. Bevorzugt enthält das Medium 4 mM oder 10 mM Pyruvat und weitere Aminosäuren.

In einer weiteren bevorzugten Ausführungsform beträgt die Startzellzahl der permanenten humanen Zellen bei Kultivierung im Schüttelkolben 5 x 10⁵ Zellen/ml, mehr bevorzugt 8 x 10⁵ Zellen/ml.

In einer weiteren bevorzugten Ausführungsform liegt der pH-Wert der Zellkultur zum Infektionszeitpunkt im Bereich von 7,1 bis 7,8, mehr bevorzugt im Bereich von 7,3 bis 7,5, noch mehr bevorzugt im Bereich von 7,3 bis 7,5.

In einer weiteren bevorzugten Ausführungsform der vorliegenden Erfindung wird vor der Infektion der humanen permanenten Zellen mit Influenzavirus ein kompletter Mediumwechsel oder eine 1:2-Verdünnung mit Medium durchgeführt.

In einer bevorzugten Ausführungsform der vorliegenden Erfindung wird zur Infektion der humanen permanenten Zellen mit Influenzavirus Trypsinkonzentrationen von 1 x 10⁻⁴ U/Zelle, 1 x 10⁻⁵ U/Zelle, 3 x 10⁻⁵ U/Zelle, 5 x 10⁻⁵ U/Zelle oder 1 x 10⁻⁶ U/Zelle verwendet. Wird vor der Infektion der humanen permanenten Zellen kein Mediumwechsel durchgeführt, wird bevorzugt eine Trypsinkonzentration von 1 x 10⁻⁴ U/Zelle bei der Infektion der Zellen mit Influenzavirus verwendet. Wird vor der Infektion der humanen permanenten Zellen eine 1:2-Verdünnung mit Medium durchgeführt, wird bevorzugt eine Trypsinkonzentration von 5 x 10⁻⁵ U/Zelle bei der Infektion der Zellen mit Influenzavirus verwendet. Wird vor der Infektion der humanen permanenten Zellen ein kompletter Mediumwechsel durchgeführt, wird bevorzugt eine Trypsinkonzentration von 5 x 10⁻⁶ U/Zelle bei der Infektion der Zellen mit Influenzavirus verwendet.

In einer bevorzugten Ausführungsform der vorliegenden Erfindung wird zur Infektion der permanenten humanen Zellen eine Virusmenge angegeben als MOI- (mulitplicity of infection) Wert im Bereich von 0,001 bis 0,3 verwendet. In einer bevorzugten Ausführungsform wird zur Infektion der permanenten humanen Zellen eine Virusmenge angegeben als MOI- (mulitplicity of infection) Wert von 0,25, 0,1, 0,06, 0,025 oder 0,0025 verwendet. Bevorzugt wird bei Infektion der permanenten humanen Zellen mit Influenzavirus A/PR/8/34 ohne Durchführung eines Mediumwechsels vor Infektion eine Virusmenge angegeben als MOI-Wert von 0,25 verwendet, bei Infektion der humanen permanenten Zellen mit Influenzavirus A/Brisbane/59/2007 ohne Durchführung eines Mediumwechsels vor Infektion wird eine Virusmenge angegeben als MOI-Wert von 0,1 verwendet. Bevorzugt wird bei Infektion der permanenten humanen Zellen mit Influenzavirus A/PR/8/34 mit Durchführung eines Mediumwechsels vor Infektion eine Virusmenge angegeben als MOI-Wert von 0,1 oder 0,25 verwendet, bei Infektion der permanenten humanen Zellen mit Influenzavirus A/Brisbane/59/2007 mit Durchführung eines Mediumwechsels vor Infektion ein MOI-Wert von 0,06 oder 0,25 und bei Infektion der permanenten humanen Zellen mit Influenzavirus B/Florida/4/2006 mit Durchführung eines Mediumwechsels vor Infektion wird eine Virusmenge angegeben als MOI-Wert von 0,1, 0,025 oder 0,0025 verwendet.

In einer bevorzugten Ausführungsform liegt die Zellzahl zum Zeitpunkt der Infektion bei Kultivierung im Schüttelkolben in einem Bereich von 1 x 10⁶ bis 6 x 10⁶ Zellen/ml. Bevorzugt beträgt die Zellzahl zum Infektionszeitpunkt 2,3 x 10⁶ Zellen/ml, 4,5 x 10⁶ Zellen/ml oder 5 x 10⁶ Zellen/ml. In einer bevorzugten Ausführungsform der vorliegenden Erfindung ist die Zellzahl zum Zeitpunkt der Infektion 4,5 x 10⁶ Zellen/ml und es wird vor Infektion kein Mediumwechsel durchgeführt. In einer weiteren bevorzugten Ausführungsform ist die Zellzahl zum Zeitpunkt der Infektion 2,3 x 10⁶ Zellen/ml und es wird vor Infektion eine 1:2-Verdünnung mit frischem PEM-Medium durchgeführt. In einer weiteren bevorzugten Ausführungsform ist die Zellzahl zum Zeitpunkt der Infektion 5 x 10⁶ Zellen/ml und es wird vor Infektion ein kompletter Mediumwechsel durchgeführt.

In einer besonders bevorzugten Ausführungsform werden die permanenten humanen Zellen im 1 Liter Bioreaktor STR (Sartorius) in PEM-Medium mit 4 mM Glutamin und 4 mM Pyruvat kultiviert, wobei die Startzellzahl 5 x 10⁵ Zellen/ml beträgt und wobei bei einer Zellzahl von 2,1 x 10⁶ Zellen/ml mit Influenzavirus in einer Virusmenge angegeben als MOI-Wert von 0,025 infiziert wird und vor Infektion kein Mediumwechsel stattfindet. Bevorzugt erfolgt die Infektion in Anwesenheit von Trypsin in einer Endkonzentration von 3 x 10⁻⁵ U/ml.

In einer besonders bevorzugten Ausführungsform werden die permanenten humanen Zellen im 1 Liter Bioreaktor STR (Sartorius) in PEM-Medium kultiviert, wobei die Startzellzahl 8 x 10⁵ Zellen/ml beträgt und wobei mit Influenzavirus unter Verwendung einer Virusmenge angegeben als MOI-Wert von 0,025 infiziert wird und vor Infektion ein Mediumwechsel stattfindet. Bevorzugt erfolgt die Infektion in Anwesenheit von Trypsin in einer Endkonzentration von 3 x 10⁻⁵ U/ml.

In einer weiteren besonders bevorzugten Ausführungsform werden die permanenten humanen Zellen im 1 Liter Wave Bioreaktor (Wave Biotech AG) in PEM-Medium mit 4 mM Glutamin, 4 mM Pyruvat und 20 mM Glucose in PEM-Medium kultiviert, wobei die Startzellzahl 5 x 10⁵ Zellen/ml beträgt und wobei die Zellzahl vor Infektion 2,1 x 10⁶ Zellen/ml beträgt und mit Influenzavirus unter Verwendung einer Virusmenge angegeben als MOI-Wert von 0,025 infiziert wird und vor Infektion kein Mediumwechsel stattfindet. Bevorzugt erfolgt die Infektion in Anwesenheit von Trypsin in einer Endkonzentration von 3 x 10⁻⁵ U/ml.

In einer bevorzugten Ausführungsform werden die permanenten humanen Zellen in PEM-Medium mit 4 mM Glutamin und 4 mM Pyruvat im Schüttelkolben kultiviert, wobei vor Infektion der Zellen mit Influenzavirus unter Verwendung einer Virusmenge angegeben als MOI-Wert von 0,025 in Anwesenheit einer Trypsin-Konzentration von 1 x 10⁻⁶ U/Zelle ein Mediumwechsel durchgeführt wird.

In einer bevorzugten Ausführungsform werden die permanenten humanen Zellen in PEM-Medium mit 4 mM Glutamin und 4 mM Pyruvat im Schüttelkolben kultiviert, wobei vor Infektion der Zellen mit Influenzavirus unter Verwendung einer Virusmenge angegeben als MOI-Wert von 0,025 in Anwesenheit einer Trypsin-Konzentration von 1 x 10⁻⁵ U/Zelle ein 1:1-Mediumwechsel durchgeführt wird.

Bei der Herstellung von Influenza-Proteinen und Nukleinsäuremolekülen kodierend für ein Influenza-Protein werden die kultivierten humanen Zellen mit Nukleinsäuremolekülen kodierend für ein Influenza-Protein transfiziert und anschließend das Influenzavirus-Protein oder die Nukleinsäuremoleküle kodierend für ein Influenza-Protein unter Verwendung bekannter Verfahren isoliert und aufgereinigt.

In einer weiteren Ausführungsform befinden sich die humanen Zellen in oder zwischen der mittleren exponentiellen Wachstumsphase und der stationären Wachstumsphase im erfindungsgemäßen Verfahren zum Zeitpunkt der Infektion mit einem Viruspartikel oder zum Zeitpunkt der Transfektion mit einem Nukleinsäuremolekül kodierend für ein Influenzavirus-Protein oder Teil davon. Eine typische Wachstumskurve in der die Zellanzahl gegen die Zeit aufgetragen wird, weist einen sigmoidalen Kurvenverlauf auf. Sie beginnt mit einer so genannten lag-Phase, gefolgt von der log-Phase oder exponentiellen Phase und der stationären Phase. Die mittlere exponentielle Wachstumsphase entspricht hierbei dem ersten Wendepunkt einer typischen Wachstumskurve, wobei ein Wendepunkt ein Punkt auf der Wachstumskurve ist, an dem sich die Form des Kurvenverlaufs von konkav zu konvex oder von konvex zu konkav ändert. Die stationäre Phase beginnt, wenn die Wachstumskurve ein Plateau erreicht und somit die Zellanzahl konstant bleibt.

Die mit dem Verfahren hergestellten Nukleinsäuren kodierend für ein Influenza-Protein können zur Nukleinsäuren-Immunisierung oder als sogenannter DNA-Impfstoff verwendet werden. Bei der Nukleinsäuren-Immunisierung werden immunogene Antigene, d.h. Antigene die eine Immunantwort im Menschen hervorrufen, inokuliert. Diese immunogenen Antigene werden durch DNA oder RNA kodiert und liegen als Expressionskassetten oder Expressionsvektoren vor oder sind in virale Vektoren integriert, um eine Immunantwort auf das Genprodukt zu induzieren. DNA-Impfstoffe können in unterschiedlichen Verabreichungssystemen vorliegen, z.B. als DNA oder RNA, in Form linearisierter oder zirkulärer Plasmide oder Expressionskassetten, wobei diese mit den zur Expression nötigen Elemente, wie z.B. Promotor, Polyadenylierungsstellen, Replikationsursprung, etc. ausgestattet sind. Bei einer Verabreichung von DNA, liegt diese meist in einem Puffer mit oder ohne Adjuvant oder an Nanopartikel gebunden oder in einer Adjuvant enthaltenden Verbindung oder in einen viralen oder bakteriellen Vektor integriert vor. DNA-Impfstoffe rufen sowohl humorale als auch Zell-vermittelte Immunität hervor. Ein Vorteil des DNA-Impfstoffs ist, dass das Antigen in seiner nativen Form exprimiert wird und somit zu einer verbesserten Immunisierung führt. Ein weiterer Vorteil des DNA-Impfstoffs ist, dass dieser im Gegensatz zu abgeschwächtem Lebendimpfstoff nicht infektiös ist und auch nicht wieder virulent werden kann.

Die Verabreichung des DNA-Impfstoffs in Form von DNA oder RNA, von Plasmiden oder linearisierten DNA-Fragmenten, die an Partikel gekoppelt sind, kann durch Injektion oder mit Hilfe einer Genegun erfolgen. Dabei kann der DNA-Impfstoff für die Injektion z.B. in einer Kochsalz- oder gepufferter Kochsalzlösung vorliegen.

Die mit dem Verfahren hergestellten Nukleinsäuren kodierend für Influenza-Proteine, Influenza-Proteine und Influenzavirus können als Impfstoff gegen Influenzavirus-Typ-A und/oder -B und/oder -C eingesetzt werden.

Der mit dem erfindungsgemäßen Verfahren hergestellte Influenzavirus-basierte Impfstoff umfasst sämtliche Proteine, Peptide oder Teile davon, sowie Nukleinsäuren kodierend für diese Proteine, Peptide oder Teile davon des Influenzavirus, sowie Influenzaviruspartikel selbst, rekombinante Influenzavirus-Proteine, einschließlich Influenza-Hüllproteine, subvirale Partikel, Virus-ähnliche Partikel (VLP), VLP-Komplexe, und/oder Teile davon, die zu Immunisierungszwecken gegen Influenza verwendet werden können.

Bevorzugt handelt es sich bei dem mit dem erfindungsgemäßen Verfahren hergestellten Influenza-Proteinen um Proteine oder Derivate des Influenzavirus, bevorzugt der Influenzavirusstämme A/PR/8/34, A/Uruguay/716/2007, A/Brisbane/59/2007, B/Florida/4/2006, Schweineinfluenza (A/Swine (H1N2) Bakum/1832/00) oder Pferdeinfluenza (A/Equine, A/Newmarket/1/93 (H3N8)).

Die Isolierung und Aufreinigung der mit dem Verfahren hergestellten Nukleinsäuren kodierend für ein Influenzavirus-Protein oder Teil davon erfolgt mit den gängigen Methoden und sind dem Fachmann bekannt.

Die Isolierung und Aufreinigung der mit dem erfindungsgemäßen Verfahren hergestellten Influenzavirus-Proteinen erfolgt mittels Verfahren, die dem Fachmann bekannt sind. Die Aufbereitung der Proteine ist zunächst abhängig von ihrer Herkunft. Man unterscheidet zwischen intra- und extrazellulären Proteinen. Befinden sich die Proteine innerhalb von Zellkörpern ist zunächst ein Aufschluss der Zellen nötig z.B. durch Scherkräfte oder Osmolyse. Danach erfolgt die Abtrennung von unlöslichem Material, wie zum Beispiel Zellmembranen und Zellwände z.B. durch Zentrifugieren. Die Zentrifugation wird standardmäßig zur Trennung von Zellen, Zellorganellen und Proteinen verwendet. Ein effektiveres Verfahren hinsichtlich der Trennkapazität ist die Pulselektrophorese. Zusätzlich gibt es nach der Abtrennung anderer Zellbestandteile auch noch die Notwendigkeit der Trennung verschieden großer Proteine, Peptide und Aminosäuren. Die Abtrennung von Proteinen kann durch ein- oder zweidimensionale Gelelektrophorese oder Kapillarelektrophorese erfolgen. Im Bereich der Aminosäuren und Peptide werden beispielsweise chromatographische Verfahren, wie Affinitäts-, Ionenaustausch- (IEC) oder Reversed-Phase-Chromatographie (RPC) verwendet. Nachteilig wirken sich auf die Aufreinigung die Anwesenheit von Lipiden und die Notwendigkeit der Abtrennung oder Deaktivierung von Proteasen aus. In der extrazellulären Matrix vorkommende Proteine müssen nicht aus Zellen extrahiert werden, liegen aber nach Abtrennung aller unlöslichen Bestandteile sehr verdünnt und gewöhnlich in deutlich geringeren Mengen als intrazelluläre Proteine vor.

Für die Isolierung und Aufreinigung der mit dem Verfahren hergestellten Influenzavirus-Partikeln werden Verfahren verwendet, die dem Fachmann bekannt sind. Beispiele für diese Verfahren sind die Dichtegradienten-Differential- oder Zonenzentrifugation.

Die im erfindungsgemäßen Verfahren verwendeten permanenten humanen Zellen entstehen durch Immortalisierung von primären humanen Zellen. Primäre humane Zellen werden durch direkte Entnahme aus dem Organismus oder einem dem Organismus entnommenen Gewebe erhalten und in Kultur genommen. Bevorzugt sind solche primären humanen Zellen, die durch Expression mit Zell-transformierenden Faktoren gut in permanente humane Zelllinien umgewandelt werden können, insbesondere Amniozyten, embryonale Retinazellen sowie embryonale Zellen neuronalen Ursprungs.

Zell-transformierende Faktoren können T-Antigen von SV40 (Genbank Acc. Nr. J02400), E6- und E7-Genprodukt von HPV (z.B. HPV16, Genbank Acc. Nr. K02718) und E1A- und E1B-Genprodukte von humanen Adenoviren (z.B. humanes Adenovirus Serotyp-5, Genbank Acc. Nr. X02996). Die primären Zellen werden zur Immortalisierung durch die Expression von E1 des humanen Adenovirus mit den beiden Nukleinsäuresequenzen für die E1A- und E1B-Genprodukte transfiziert. Bei der Expression durch ein natürlicherweise vorliegendes HPV können E6 und E7 von einem RNA-Transkript exprimiert werden. Gleiches gilt für die Expression von E1A und E1B eines natürlicherweise vorliegenden Adenovirus. Die Zell-transformierenden Faktoren wie z.B. die adenovirale E1-Genfunktion bewirken die Immortalisierung oder Transformation und somit die dauerhafte Kultivierbarkeit der Zellen.

Die Expression der Zell-transformierenden Faktoren kann unter Kontrolle eines homologen Promotors bzw. transkriptioneller Terminationselemente erfolgen, z.B. den natürlichen E1A-Promotor und die natürliche E1A-Polyadenylierungsstelle für die Expression der adenoviralen E1A-Genfunktion. Dies kann erreicht werden, indem die für die Transfektion benutzten Nukleinsäuremoleküle Fragmente des jeweiligen viralen Genoms, z.B. des adenoviralen Genoms, mit den genannten Genfunktionen, z.B. E1A, E1B, enthalten. Die Expression der Zell-transformierenden Faktoren kann aber auch unter der Kontrolle heterologer, nicht natürlicherweise mit der verwendeten codierenden Region vorkommenden Promotoren oder transkriptionellen Terminationselementen erfolgen. Als heterologe Promotoren können z.B. CMV- (Cytomegalievirus-) Promotor (Makrides, 9-26 in: Makrides (Hrsg.), Gene Transfer and Expression in Mammalian Cells, Elsevier, Amsterdam, 2003), EF-1α-Promotor (Kim et al., Gene 91:217-223, 1990), CAG-Promotor (ein Hybridpromotor aus dem Immediate Early-Enhancer des humanen Cytomegalievirus und einem modifizierten Huhn β-Aktin Promotor mit erstem Intron) (Niwa et al., Gene 108:193-199, 1991), humaner oder muriner pgk-(Phosphoglyceratkinase-)Promotor (Adra et al., Gene 60:65-74, 1987), RSV- (Rous Sarkoma Virus-) Promotor (Makrides, 9-26 in: Makrides (Hrsg.), Gene Transfer and Expression in Mammalian Cells, Elsevier, Amsterdam, 2003) oder SV40- (Simian Virus 40-) Promotor (Makrides, 9-26 in: Makrides (Hrsg.), Gene Transfer and Expression in Mammalian Cells, Elsevier, Amsterdam, 2003) dienen. Als Polyadenylierungsstellen können z.B. die Polyadenylierungssequenzen des SV40 Large T-Antigens (Genbank Acc. Nr. J02400) oder des humanen G-CSF- (Granulozyten-Kolonie-stimulierender Faktor, granulocyte colony stimulating factor) Gens (Mizushima und Nagata, Nucl. Acids Res. 18:5322, 1990) dienen.

Durch die Transfektion der primären humanen Zellen mit dem Nukleinsäuremolekül umfassend die für E1A- und E1B-kodierenden Nukleinsäuresequenzen, werden die Zellen immortalisiert. Das zur Immortalisierung der primären humanen Zellen verwendete Nukleinsäuremolekül umfasst E1A- und E1B-Nukleinsäuresequenzen, die vorzugsweise von humanen Adenoviren stammen, insbesondere vom humanen Adenovirus Serotyp-5. In einer bevorzugten Ausführungsform umfasst das zur Immortalisierung verwendete Nukleinsäuremolekül, neben den E1A und E1B-kodierenden Nukleinsäuresequenzen, die Nukleinsäuresequenz kodierend für die adenovirale pIX-Genfunktion. Das pIX-Polypeptid, ein virales Strukturprotein, wirkt als Transkriptionsaktivator auf verschiedene virale und zelluläre Promotoren wie z.B. den Thymidinkinase- und den Beta-Globin-Promotor. Die transkriptionsaktivierende Wirkung des zusätzlich in der Zelle exprimierten pIX-Polypeptids kann in den erfindungsgemäßen Produktionszelllinien zu einer Erhöhung der Expressionsraten des rekombinanten Polypeptids führen, falls die codierende Sequenz des rekombinanten Polypeptids unter der Kontrolle eines der zuvor genannten Promotoren steht. Eine beispielhafte Sequenz findet sich in Genbank Acc. Nr. X02996. Insbesondere umfassen die Nukleinsäuremoleküle die Nukleotide 1 bis 4344, 505 bis 3522 oder die Nukleotide 505 bis 4079 des humanen Adenovirus Serotyp-5.

In einer bevorzugten Ausführungsform enthält das Nukleinsäuremolekül zur Immortalisierung der primären Zellen, insbesondere der Amniozyten die Adenovirus Serotyp 5 Nukleotidsequenz von Nukleotid 505 bis Nukleotid 4079. In einer weiteren besonders bevorzugten Ausführungsform enthält das Nukleinsäuremolekül zur Immortalisierung der primären Zellen, insbesondere der Amniozyten die Adenovirus Serotyp-5-Nukleotidsequenz von Nukleotid 505 bis Nukleotid 3522. In einer weiteren besonders bevorzugten Ausführungsform enthält das Nukleinsäuremolekül zur Immortalisierung der primären Zellen, insbesondere der Amniozyten die Adenovirus Serotyp-5-Nukleotidsequenz von Nukleotid 1 bis Nukleotid 4344, die der adenoviralen DNA in HEK293-Zellen entspricht (Louis et al., Virology 233:423-429, 1997). Ferner kann die immortalisierte humane Zelle einen viralen Replikationsfaktor exprimieren. Dieser Replikationsfaktor kann an den Replikationsursprung (ori, "origin of replication") eines durch Transfektion eingebrachten Nukleinsäuremoleküls binden und dadurch die Replikation des episomalen Nukleinsäuremoleküls initiieren. Die episomale Replikation von Nukleinsäuremolekülen, insbesondere von Plasmid-DNA, in Zellen bewirkt eine starke Vermehrung der Kopienzahl der transferierten Nukleinsäuremoleküle und dadurch eine Erhöhung der Expression eines auf diesem Molekül codierten rekombinanten Polypeptids sowie dessen Erhalt über viele Zellteilungen hinweg. Ein solcher viraler Replikationsfaktor ist z.B. das T-Antigen des Simian Virus 40 (SV40), das nach Bindung an eine als SV40-Replikationsursprung (SV40 ori, origin of replication) bezeichnete Sequenz auf dem Nukleinsäuremolekül, z.B. der Plasmid-DNA, dessen Replikation initiiert. Das Ebstein-Barr-Virus Protein EBNA-1 (Ebstein Barr Virus Nuclear Antigen-1) erkennt einen als ori-P bezeichneten Replikationsursprung und katalysiert die extrachromosomale Replikation des ori-P tragenden Nukleinsäuremoleküls. Das T-Antigen des Simian Virus 40 (SV40) aktiviert nicht nur als Replikationsfaktor die Replikation, sondern weist ebenfalls eine aktivierende Wirkung auf die Transkription einiger viraler und zellulärer Gene auf (Brady, John and Khoury, George, 1985, Molecular and Cellular Biology, Vol. 5, No. 6, p. 1391 to 1399).

Die im erfindungsgemäßen Verfahren verwendete immortalisierte humane Zelle ist insbesondere eine immortalisierte humane Amniozyten-Zelle. In einer bevorzugten Ausführungsform exprimiert die im erfindungsgemäßen Verfahren verwendete immortalisierte humane Zelle das große T-Antigen von SV40 oder das Epstein-Barr-Virus (EBV) Nuclear Antigen-1 (EBNA-1). In einer besonders bevorzugten Ausführungsform exprimiert die im erfindungsgemäßen Verfahren verwendete immortalisierte humane Amniozyten-Zelle das große T-Antigen von SV40 oder das Epstein-Barr-Virus (EBV) Nuclear Antigen-1 (EBNA-1). In einer weiteren besonders bevorzugten Ausführungsform exprimiert die im erfindungsgemäßen Verfahren verwendete immortalisierte humane Zelle, insbesondere Amniozyten-Zelle das große T-Antigen von SV40 unter Kontrolle des CAG-, RSV- oder CMV-Promotors.

Die im Verfahren der vorliegenden Erfindung verwendeten permanenten humanen Amniozyten sind insbesondere in den Patentschriften EP 1230354 und EP 1948789 beschrieben. In einer besonders bevorzugten Ausführungsform handelt es sich bei der in dem erfindungsgemäßen Verfahren verwendeten permanenten humanen Amniozytenzelle um CAP oder CAP-T.

Im Fall der CAP-Zellen wurden die primären Amniozyten mit einem Plasmid transfiziert, das den murinen pgk Promotor, Ad5-Sequenzen nt. 505-3522 enthaltend die gesamte E1-Region, das 3' Spleiß- und Polyadenylierungssignal von SV40 und die pIX-Region von Ad5 (nt. 3485-4079) enthält. Dieses Plasmid wurde bereits detailliert in EP 1 948 789 beschrieben.

Zur Herstellung der CAP-T-Zellen wurden die CAP-Zellen mit einem Plasmid transfiziert, das die Expressionskassette für T-Antigen von SV40 flankiert von einem Intron aus SV40 und einer Polyadenylierungsstelle umfasst. Zusätzlich kann das Plasmid den CAG-Promotor (Hybridpromotor bestehend aus dem CMV-Enhancer und dem Huhn β-Aktin-Promotor) (Niwa et al., Gene 108:193-199, 1991), den RSV-Promotor (Promotor aus Rous Sarcomavirus) (Genbank Acc. Nr.DQ075935) oder den CMV-Promotor (früher Promotor des humanen Cytornegalie-Virus) (SEQ ID NO:5) enthalten. Zur Generierung stabiler Zelllinien enthält das Plasmid eine Blasticidin-Expressionskassette mit dem Ubiquitinpromotor (pUB/Bsd, Invitrogen #V512-20).

Darüber kann die humane Zelle, insbesondere die Amniozytenzelle in Suspension wachsen kann. Ferner kann die humane Zelle, insbesondere die Amniozytenzelle im erfindungsgemäßen Verfahren in Serum-freiem Medium kultiviert werden.

Ein weiterer Gegenstand der vorliegenden Erfindung ist die Verwendung einer permanenten humanen Zelle, insbesondere einer Amniozytenzelle zur Herstellung eines Influenzavirusbasierten Impfstoffs.

In einer bevorzugten Ausführungsform handelt es sich bei der verwendeten permanenten humanen Amniozytenzelle zur Herstellung eines Influenzavirus-basierten Impfstoffs um eine CAP- oder CAP-T-Zelle.

Der mit dem erfindungsgemäßen Verfahren hergestellte Influenzavirus-basierte Impfstoff kann ein Influenzavirus und/oder ein Influenzavirus-Protein oder ein Nukleinsäuremolekül kodierend für ein Influenza-Protein sein. Der Impfstoff kann parenteral mit einer Spritze verabreicht werden. Man unterscheidet hierbei intradermale, subkutane oder intramuskuläre Injektionen. Die intradermale Injektion kann mit einer Impfpistole oder Lanzette erfolgen. Die intramuskuläre Injektion kann in den Oberarm, in den Oberschenkel oder in den Gesäßmuskel erfolgen. Ferner kann der Impfstoff oral oder nasal verabreicht werden. Der Impfstoff kann beispielsweise Menschen und Tieren verabreicht werden.

Der mit dem erfindungsgemäßen Verfahren hergestellte Influenzavirus-basierte Impfstoff kann sowohl durch aktive wie auch passive Immunisierung eine Resistenz gegen eine oder mehrere Influenzaviren verleihen. Bei der aktiven Immunisierung dient der Impfstoff nach Applikation zur spezifischen Aktivierung des Immunsystems von Menschen und Tieren hinsichtlich eines bestimmten Virus. Hierbei wird die Reaktion des Immunsystems ausgenutzt, bei Vorhandensein von Viren bzw. deren spezifischen Antigenen eine Immunantwort hervorzurufen. Diese führt zur Ausbildung von Antikörpern und spezialisierten T-Helferzellen, die dann einen lange anhaltenden Schutz vor der jeweiligen Krankheit bieten, der je nach Virus zwischen einigen Jahren und lebenslang anhalten kann. Bei dem mit dem erfindungsgemäßen Verfahren hergestellten Influenzavirus-basierten Impfstoff kann es sich beispielsweise um einen Lebend- oder Totimpfstoff handeln. Der Lebendimpfstoff enthält zum Beispiel abgeschwächte, noch vermehrungsfähige Viren, welche die Krankheit nicht auslösen können. Bei einem Totimpfstoff wurden diese Viren dagegen abgetötet oder er enthält nur noch Bruchstücke des Virus (Antigene). Die Inaktivierung (Abtötung) der Viren erfolgt beispielsweise durch chemische Stoffe/Stoffkombinationen, z. B. Formaldehyd, beta-Propiolacton und Psoralen. Die Virushülle bleibt dabei erhalten. Es gibt auch Toxoidimpfstoffe, die nur den biologisch inaktiven Bestandteil (Toxoid) des Toxins eines Virus enthalten (z.B. das Tetanus-Toxoid), die ebenfalls zu den Totimpfstoffen gezählt werden. Insbesondere kann es sich bei dem Totimpfstoff um einen Spaltimpfstoff handeln, der aus Bruchstücken der Virushüllproteine besteht. Die Zerstörung (Spaltung) der Virushülle kann beispielsweise mit Detergentien oder starken organischen Lösungsmitteln erfolgen. Die Viren können auch zusätzlich mit chemischen Stoffen inaktiviert (abgetötet) werden. Ferner zählen zu den Totimpfstoffen die Untereinheitimpfstoffe, die aus spezifischen Komponenten des Virus beispielsweise Hämagglutinin- und Neuraminidase-Proteine bestehen.

Bei einer passiven Immunisierung wird der mit dem erfindungsgemäßen Verfahren hergestellte Influenzavirus-basierte Impfstoff einem Wirt (z.B. einem Säuger) verabreicht, das hervorgerufenen Antiserum gewonnen und dem Empfänger, der mit mindestens einem Influenzavirus infiziert ist, verabreicht.

Ferner wird der Impfstoff zur Verabreichung mit einem oder mehreren Zusatzstoffen, wie Stabilisatoren, Neutralisatoren, Träger und Konservierungsstoffen versetzt. Zu diesen Stoffen zählen unter anderem Formaldehyd, Thiomersal, Aluminiumphosphat, Azeton und Phenol. Außerdem kann der Impfstoff mit Hilfsstoffen zur Verstärkung der Wirkung des Impfstoffs versetzt werden. Diese so genannten Adjuvantien sollten möglichst selbst keine pharmakologische Wirkung aufweisen und insbesondere als Lösungsvermittler, Emulsionen oder Mischungen daraus dienen. Adjuvantien sind zum Beispiel Mineralsalze, Squalen-Gemische, Muramyl-Peptide, Saponin-Derivate, Mycobakterien-Zellwand-Präparationen, bestimmte Emulsionen, Monophosphoryl-Lipid-A, Mykolsäure-Derivate, nichtionische Block-Copolymer-Tenside, Quil A, Untereinheit des Cholera-Toxins B, Polyphosphazene und dessen Derivate, Immunstimulierende Komplexe, Cytokin-Adjuvantien, MF59-Adjuvans, Lipid-Adjuvantien, mukosale Adjuvantien, bestimmte Bakterien-Exotoxine, bestimmic Oligonukleotide und PLG.

Die folgenden Beispiele erläutern die Erfindung Sofern nicht anders angegeben, wurden molekularbiologische Standardmethoden verwendet. wie z.B. von Sambrook et al., 1989, Molecular cloning: A Laboratory Manual 2. Auflage, Cold Spring Harbor Laboratory Press, Cold Spring Harbor, New York, beschrieben.

### Beispiel 1: Kultivierungsversuche mit der permanenten Amniozytenzelllinie CAP-1D5 in PEM- oder 293SFMII-Medium

Die permanente Amniozytenzelllinie CAP-1D5 wurde in 100 ml serumfreiem 293SFMII-Medium (Invitrogen) oder PEM-Medium (Invitrogen) bei 37°C, 8% CO₂ und 100 rpm kultiviert. Zur Kontrolle wurde die permanente Hundenierenzelllinie MDCK SUS2 (Madin Darby Canine Kidney, adaptiert an Wachstum in Suspension) (Lohr et al., Vaccine. 2010, 28(38):6256-64) in 100 ml aerumfreien SMIF8- Medium in 100 ml Schüttelkolben verwendet.

Zum Zeitpunkt 0 *(=Startpunkt der Kultur)* sowie jeweils in einer Zeitspanne von 24 h wurde die Lebendzellzahl, die Totzellzahl, die Überlebensrate der Zelllinien bestimmt, sowie der pH-Wert, die Konzentration an Glucose, Laktose, Glutamin, Ammonium, Glutaminsäure und Pyruvat im Medium (Biochemisches Multiparameter Analysesystem) (Lohr et al., Vaccine. 2009, 27(37), 4975-4982; Genzel et al., Appl Microbiot Biotechnol., 2010, 88(2):461-75).

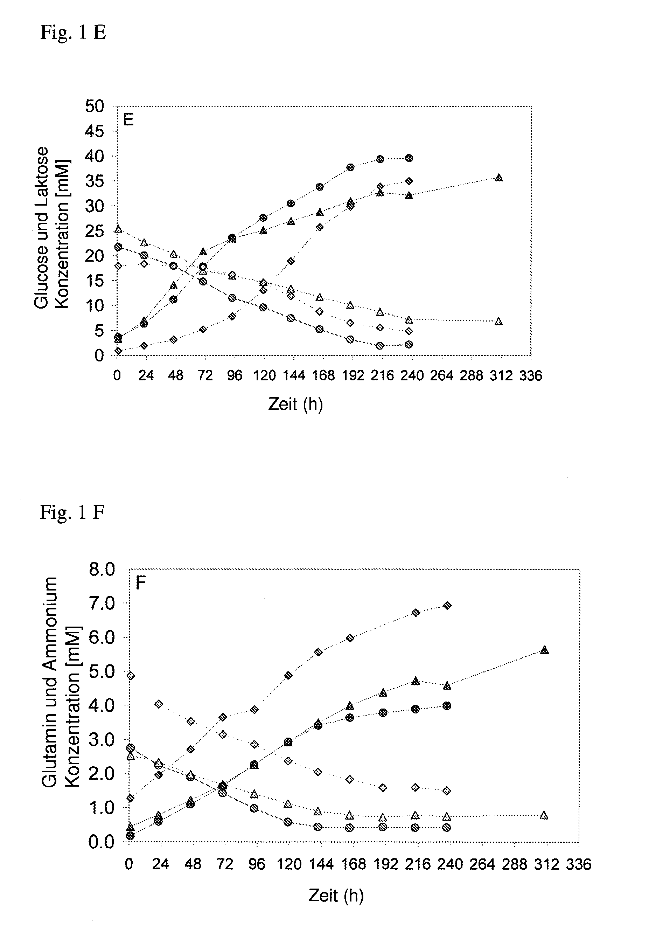

Die Ergebnisse sind in Fig. 1 dargestellt. Die Lebendzellzahlen der permanenten Amniozytenzelllinie CAP-1D5 in 293SPMII-, wie auch in PEM-Medium, als auch die MDCK.SUS2-Zellen, ausgehend von etwa 2 x 10³ Zellen pro ml Kultur zu Beginn der Kultivierung, zeigten einen ähnlichen Verlauf und erreichten nach 168 h eine Lebendzellzahl von etwa 2 x 10⁶ Zellen pro ml. Die Lebendzellzahl der MDCK. SUS2-Zellen fiel ab 192 h auf 9 x 10⁵ Zellen pro ml ab und blieb konstant bis zu 240 h nach Beginn der Wachstumskurve. Die Lebendzellzahl der permanenten Amniozytenzelllinie CAP-1D5 in 2935FMII-Medium fiel erst bei 216 h auf den Anfangswert von 2 x 10⁵ Zellen pro ml Kultur ab, hingegen blieb die Lebendzellzahl der CAP-1D3-Zellen in PEM Medium bis zu 240 h stabil auf etwa 2,5 x 10⁶ Zellen pro ml Kultur und erreichte erst nach 312 h den Anfangswert der Lebendzellzahl von 2 x 10⁵ Zellen.

Die Überlebensrate der CAP-1D5-Zellen in 293SFMII-Medium und in PEM-Medium, sowie die der MDCK.SUS2-Zellen lag zu Beginn der Wachstumskurve und bis 168 h zwischen 80 und 90 %. Die Überlebensrate der CAP-1D5-Zellen in PEM-Medium blieb bis 240 h bei 80 %, fiel dann ab und zeigte nach 312 h noch 10 %. Die Überlebensrate der CAP-1D5-Zellen in 293SFMII-Medium fiel schon nach 168 h langsam ab auf zeigte ca. 10 % nach 216 h. Die Überlebensrate der MDCK.SUS2-Zellen nahm nach 168 h stetig ab und erreicht nach 240 h etwa 45 %.

Der pH-Wert der Kultur der MDCK.SUS2-Zellen blieb über 240 h relativ stabil zwischen 7,7 und 7,6. Hingegen nahm der pH-Wert (zu Beginn 7,4) der Kultur der CAP-1D5-Zellen in 293SFMII-Medium, als auch in PEM-Medium stetig ab auf pH 6,4 nach 240 h (293SFMII-Medium) bzw. 312 h (PEM-Medium)

Die Laktose-Konzentration nahm von anfänglichen Konzentrationen von unter 5 mM im 293SFMII-Medium als auch im PEM-Medium der jeweiligen Kultur der CAP-1D5-Zellen auf 30 - 35 mM bis 240 h zu. In der Kultur der MDCK.SUS2-Zellen nahm die Laktose-Konzentration weniger stark zu, erreichte aber bei 240 h einen ähnlichen Wert wie die Kultur der CAP-1D5-Zellen. Die Glucose-Konzentration nahm 20 bis 25 mM zu Beginn der Kultur der CAP-1D5-Zellen im 293SFMII-Medium und im PEM-Medium, als auch in der Kultur der MDCK.SUS2-Zellen auf unter 10 mM ab, wobei die stärkste Abnahme in der Kultur der CAP-1D5-Zellen im 293SFMII-Medium zu verzeichnen war.

Die Zunahme der Ammonium-Konzentration in der Kultur der CAP-1D5-Zellen im 293SFMII-Medium, als auch im PEM-Medium zeigte einen sehr ähnlichen Verlauf (von <0.5 auf 4-5 mM). Im Gegensatz hierzu nahm die Ammonium-Konzentration in der Kultur der MDCK.SUS2-Zellen deutlich stärker zu (auf 7 mM). Die Abnahme der Glutamin-Konzentration verlief in der Kultur der Kultur der CAP-1D5-Zellen im 293SFMII-Medium, als auch im PEM-Medium wieder sehr ähnlich, wobei die stärkste Abnahme im PEM-Medium zu verzeichnen war.

Die höchste Glutaminsäure-Konzentration wies die Kultur der MDCK.SUS2-Zellen auf und diese stieg nur geringfügig an. Die Glutaminsäure-Konzentration war zu Beginn der Kultivierung in der Kultur der CAP-1D5-Zellen im PEM-Medium im Gegensatz zu der im 293SFMII-Medium höher und stieg stetig an. Die Pyruvat-Konzentration in der Kultur der MDCK.SUS2-Zellen nahm ab 144 h auf Null ab. Hingegen war das Pyruvat in den Kulturen der CAP-1D5-Zellen im PEM-Medium bzw. 293SFMII-Medium nach bereits 48 h aufgebraucht.

Die CAP-1D5-Zellen zeigen in PEM-Medium somit besseres Wachstum als in dem 293SFMII-Medium, Die CAP-1D5-Zellen in PEM-Medium zeigen stärksten Glucoseverbrauch und stärkste Laktose-Bildung. Die Limitierung der Glucose ab 192 h könnte den Einbruch der Zellzahl der CAP-1D5-Zellen in PEM-Medium erklären. Für die Optimierung der Kultivierungsbedingungen der Kulturen der CAP-1D5-Zellen im PEM-Medium bzw. 293SFMII-Medium könnte eine Stabilisierung des pH-Werts, die Zugabe von Pyruvat und Glutamin, sowie Glucose relevant sein.

### Beispiel 2: Virusinfektion mit nicht-adaptierten Viren

Für die Infektionsversuche mit nicht-adaptierten Viren wurde die Amniozytenzelllinie CAP-1D5 wurde in 55 ml serumfreiem 293SFMII-Medium (Invitrogen) und PEM-Medium (Invitrogen) bei 37°C, 8% CO₂, 100 rpm im Schüttelkolben kultiviert. Zur Kontrolle wurde die Hundenierenzelllinie MDCK.SUS2 (Madin Darby Canine Kidney, adaptiert an Suspension) verwendet.

Die jeweils in Tabelle 1 aufgelisteten Zelldichten wurden mit dem Virus B/Florida/4/2006 (NIBSC, The National Institute for Biological Standards and Control) bzw. A/PR/8/34 (H1N1) (RKI, Robert-Koch-Institut) ohne Mediumwechsel und mit Zugabe von 5 x 10⁻⁶ U/mL Trypsin infiziert. Die Menge an produziertem Viruspartikeln wurde durch Titration des Hämagglutinin (HA, Hämagglutinationstest nach Standardverfahren) bestimmt (dargestellt in log HA-Einheiten /100 µl; Tabelle 1) (Kalbfuss et al., 2008; Biologicals 36(3):145-61). Ebenfalls in Tabelle 1 aufgeführt sind die jeweiligen pH-Werte des Mediums zum Zeitpunkt.

**Tabelle 1: Übersicht der Virusinfektionsversuche mit nicht adaptierten Viren bei Infektion ohne Mediumwechsel**

| **Zelle** | **Medium** | **Virus** | **Lebendzellzahl/mL bei 0 hpi*** | **pH bei HAₘₐₓ** | **HAₘₐₓ** |
|---|---|---|---|---|---|
| 1D5 | 293SFM | B/Florida | 2.70E+06 | 6.6 | 0.87 |
| 1D5 | PEM | B/Florida | 2.40E+06 | 6.6 | 0 |
| 1D5 | 293SFM | A/PR/8/34 | 2.70E+06 | 6.6 | 1.19 |
| 1D5 | PEM | A/PR/8/34 | 2.50E+06 | 6.6 | 0 |
| MDCKsus | SMIF8 | B/Florida | 1.50E+06 | 7.5 | 0 |
| MDCKsus | SMIF8 | A/PR/8/34 | 1.60E+06 | 7.7 | 1.84 |

| | | | | | |
|---|---|---|---|---|---|
| • hpi: Stunden nach Infektion | | | | | |

Für die permanente Amniozytenzelllinie CAP-1D5 konnte bei Kultivierung in PEM-Medium weder bei Infektion mit dem Virus B/Florida/4/2006 (B/Florida), noch mit dem Virus A/PR/8/34 die Produktion von Viruspartikeln nachgewiesen werden. Der maximale HA-Wert für A/PR/8/34 (H1N1) in 293SFM-Medium lag unter dem in MDCK.SUS2 erreichten maximalen HA-Wert. Der pH-Wert bei maximalen HA war bei allen Infektionen in CAP-1D5 vergleichbar (pH 6,6), war jedoch deutlich geringer als in infizierten MDCK.SUS2-Zellen (pH 7,7).

Somit fand eine Infektion der permanenten Amniozytenzelllinie CAP-1D5 unter den getesteten Bedingungen lediglich mit dem Virus B/Florida statt. Im Falle der permanenten Hundenierenzelllinie MDCK.SUS2 (Madin Darby Canine Kidney) konnte hingegen nur für den Virus A/PR/8/34 (H1N1) eine Infektion nachgewiesen werden. In den nachfolgenden Experimenten sollte getestet werden, ob durch eine vorausgehende Adaptation der Viren an CAP-1D5-Zellen eine verbesserte Infektion und somit höhere Virusausbeute erreicht werden konnte.

### Beispiel 3: Virusadaptation an CAP-1D5-Zellen in PEM- und 293SFMII-Medium im Schüttelkolben

Die Virusadaptation der Influenzaviren A/PR/8/34 (H1N1) (RKI, Robert-Koch-Institut), A/Uruguay/716/2007 (H3N2) (NYMC X-175C, NIBSC, The National Institute for Biological Standards and Control) bzw. B/Florida/4/2006 (NIBSC, The National Institute for Biological Standards and Control) erfolgte durch Infektion von CAP-1D5 über 4 Passagen in Schüttelkolben in PEM- und 293SFMII-Medium. Vor jeder Infektion wurde ein Mediumwechsel durchgeführt. Die Virusausbeute während den einzelnen Passagen wurde durch Titration des Hämagglutinin (log HA-Einheiten /100 µl) und durch den Tissue Culture Infectious Dose₅₀ Assay (TCID₅₀, Virenzahl/ml) quantifiziert (Genzel and Reichl, Vaccine production - state of the art and future needs in upstream processing in Methods in Biotechnology: Animal Cell Biotechnology - Methods and Protocols; Eds. R. Pörtner; HUMANA Press Inc., Totowa, New Jersey, 2007, 457-473; Kalbfuss et al., 2008; Biologicals 36(3):145-gel).

Die Ergebnisse sind in Fig. 2 dargestellt. Die Infektion der CAP-1D5-Zellen mit dem Influenzavirus A/PR/8/34 (HIN1) und A/Uruguay/716/2007 (H3N2) führte sowohl im PEM-. als auch im 293SFMII-Medium nach 4 Passagen zu deutlich gesteigerten Virustitem. Die Infektion der CAP-1D5-Zellen mit dem Influenzavirus B/Florida/4/2006 führte sowohl bei Kultivierung im 293SFMII-Medium, als auch im PEM-Medium zu einer deutlichen Erhöhung des Virentiters in der zweiten Passage. Vergleichbare Zunahme zeigte auch die Titration des HA-Wertes bei Infektion der CAP-1D5- Zellen mit dem Influenzavirus A/PR/8134 (H1N1) bzw. A/Uruguay/716/2007 (H3N2) sowohl in PEM-. als auch in 293SFMII-Medium.

Neben einer Erhöhung des Virustiters über die Adaptation wurde auch mit jeder Passage die Replikation des Virus schneller und es konnte letztlich deutlich der Virustiter gesteigert werden. Generell scheinen in 293SFMII-Medium leicht erhöhte Virustiter im Vergleich zum PEM-Medium erreicht werden.

### Beispiel 4: MOI (multiplicity of infection)-Abhängigkert der Infektion von CAP-1D5- und MDCK.SUS2-Zellen mit adaptiertem Influenza A/PR/8/34

Mit adaptiertem Influenzavirus A/PR/8/34 (H1N1; RKI, Robert-Koch-Institut) wurde nun die MOI-Abhängigkeit bei 3 verschiedenen Werten fUr MOI (multiplicity of infection), der Anzahl der Virenpartikel pro Wirtszelle, 0,0025. 0,025 und 0,25 überprüft. Die permanenten Amniozytenzellen CAP-1D5 wurden in 293SFMII- bzw. PEM-Medium und die permanenten Hundenierenzellen MDCK.SUS2 in SMIFB-Medium in Schüttelkolben mit verschiedenen MOI des adaptierten Influenzavirus A/PR/8/34 infiziert. Bei Infektion wurde außerdem ein Mediumwechsel vorgenommen, der für nahezu konstante pH-Werte um pH=7,5 sorgte. Anschließend wurde über 96 h hinweg die Lebendzellzahl und über 144 h hinweg die Menge der Viruspartikel (log HA-Einheiten/100 µl) durch Titration des Hämagglutinin im Hämagglutinationstest nach Standardvedahren bestimmt.

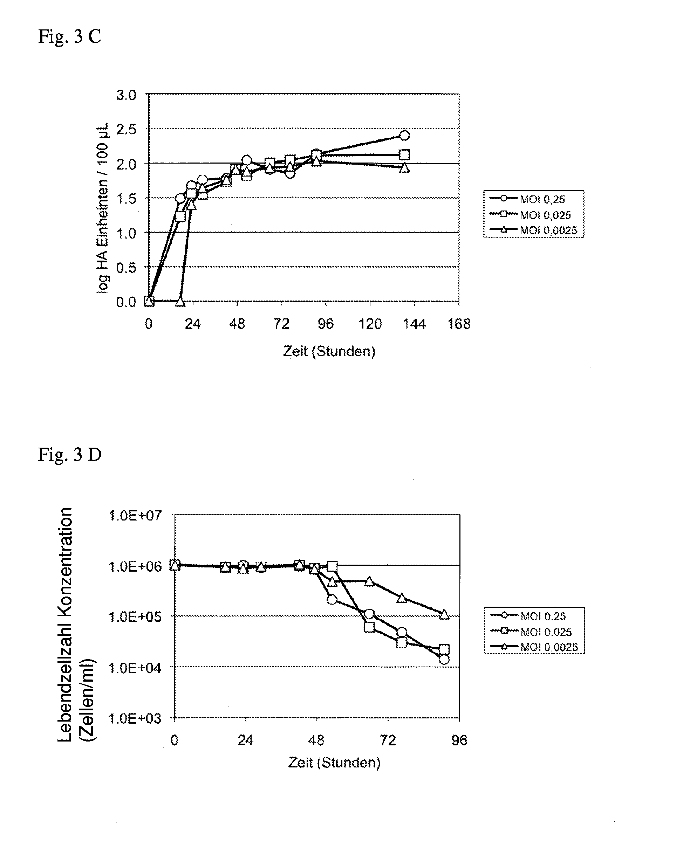

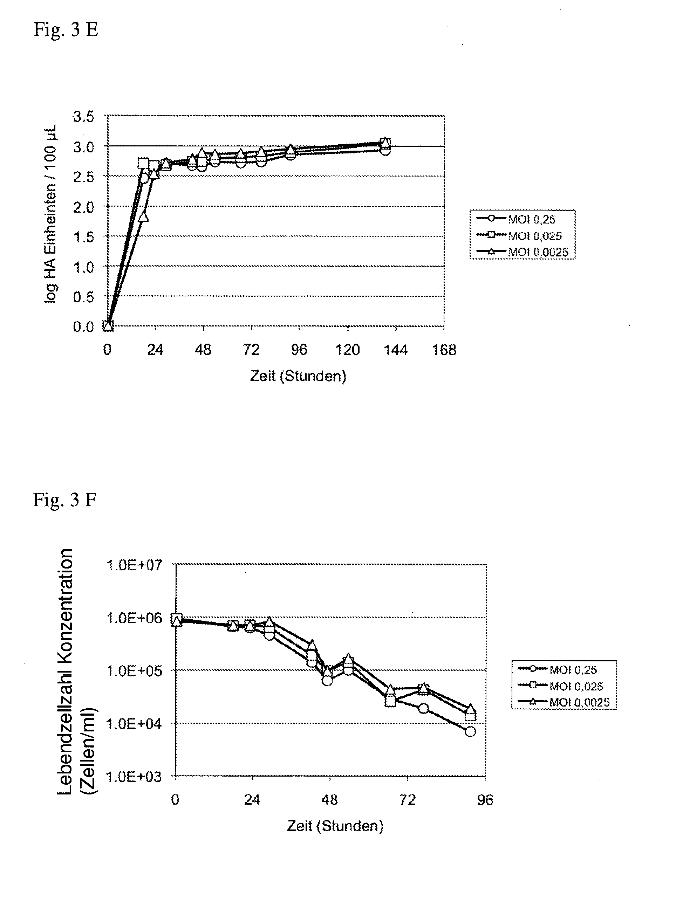

Die Ergebnisse sind in Fig. 3 dargestellt. Der Verlauf der Lebendzellzahl, sowie der Verlauf der Menge an gebildeten Viruspartikeln in der Kultur der CAP-1D5-Zellen in 293SFM-Medium und der Kultur der MDCK.SUS2-Zellen in SMIF8-Medium zeigte keine Abhängigkeit von den MOI-Werten. Der Virustiter stieg auf über 2,5 log RA-Einheiten/100 µl in den beiden Kulturen. Die Kultur der CAP-1D5-Zellen in PEM-Medium wies für alle drei MOI-Werte geringere Mengen (ca. 2,0 log HA-Einheiten/100 µl) an Viruspartikeln auf. Entsprechend dazu blieb die Lebendzellzahl der Kultur der CAP-1D5- Zellen in PEM-Medium über 48 h konstant bei 1 x 10⁶ Zellen/ml und fiel dann auf unter 1x10⁴ Zellen/ml ab. Im Gegensatz dazu fiel die Lebendzellzahl in den CAP-1D5-Zellkulturen in 293SFM- Medium und der MDCK.SUS2-Zellkultur konstant ab von 1 x 10⁶ Zellen/ml auf unter 1 x 10⁴ Zellen/ml nach 96 h. In allen Kulturen war bei einem MOI von 0,0025 eine leichte Verzögerung der Virusreplikation sichtbar, nachweisbar durch einen zeitlich verzägerte Anstieg der log HA-Einheiten im Vergleich zu den höheren MOI Werten.

### Beispiel 5: Kultivierung im 1 L Meßstab mit Infektion (A/PR/8/34 adaptiert)

Die CAP-1D5-Zellen wurden im 1 Liter Bioreaktor in PEM-Medium mit 4 mM Glutamin und 4 mM Pyruvat bei 85 rpm, pH = 7,2 und einem Sauerstoffpartialdruck pO₂ von 40% mit reinem Sauerstoff kultiviert. Die Startzellzahl betrug 5 x 10⁵ Zellen/mL.

Nach 114 h Wachstum und einer Zellzahl von 2,4 x 10⁶ Zellen/ml wurden die CAP-1D5-Zellen mit Influenzavirus A/PR/8/34 (adaptiert: in PEM, 4. Passage, 1,78 x 10⁷ Viren/mL) infiziert. Dabei wurde kein Mediumwechsel vorgenommen, aber 80 ml PEM-Medium, sowie Glutamin und Pyruvat in einer Endkonzentration von jeweils 2 mM zugegeben. Der MOI Wert betrug 0,025 und es wurde Trypsin in einer Endkonzentration von 1 x 10⁻⁵ U/mL zugegeben. Über 240 h hinweg wurde in einer Zeitspanne von jeweils 24 h die Lebendzellzahl, die Totzellzahl, die Überlebensrate der Zelllinien bestimmt, sowie der pH-Wert, die Konzentration an Glucose. Laktose, Glutamin. Ammonium, Glutaminsäure und Pyruvat im Medium. Weiter wurden ab dem Zeitpunkt der Infektion (114 h) die log HA-Einheiten/100 µl und TCID₅₀-Werte ermittelt (Genzel and Reicht. Vaccine production - state of the art and future needs in upstream processing in Methods in Biotechnology: Animal Cell Biotechnology - Methods and Protocols ; Eds. R. Pönner; HUMANA Press Inc., Totowa, New Jersey, 2007, 457-473; Kalbfuss et al.. 2008; Biologicals 36(3):145-61).

Die Ergebnisse sind in Fig. 4 dargestellt. Die Lebendzellzahl der CAP-1D5-Zellen nahm zunächst bis zur Injektion mit dem Influenza virus A/PR/8/34 von 6 x 10⁵ Zellen/ml auf 2,4 x 10⁶ Zellen/ml zu und nach der Infektion wieder etwas ab. Die Überlebensrate der CAP-ID5-Zellen lag über die gesamte Zeit hinweg zwischen 80 und 90 % und nahm erst nach 240 h auf 70 % ab. Die Pyruvat-Konzentration in der Kultur nahm innerhalb von 72 h auf Null ab, die zugegebene Menge an Pyruvat bei der Infektion mit dem Influenzavirus war ebenfalls innerhalb von 10 h aufgebraucht. Die Glutamat-Konzentration stieg über die gesamte Zeit stetig von etwa 1 mM auf etwa 1,8 mM an. Der pH-Wert der Kultur war über die beobachtete Zeit bis auf wenige Schwankungen bei 7,1 bis 7.4. Der maximal erreichte TCID₅₀-Titer lag bei 2.4 x 10⁷ Viren/mL, der maximale HA-Titer lag bei 2,2 log HA-Einheiten/100µl.

Die Ergebnisse dieses Wachstumsexperiments zeigen, dass aufgrund der Zufütterung von Pyruvat keine Glucoselimitierung auftrat und somit die Lebendzellzahl nicht einbricht

### Beispiel 6: Virusadaptation an CAP-1D5 Zellen in PEM- und 293SFMII-Medium in 50 ml-Falcons

Die Virusadaptation der lnfluenzaviren A/Brisbane/59/2007 (HIN1-like HGR; IVR-148, NIBSC. The National Institute for Biological Standards and Control), B/Florida/4/2006 (NIBSC. The National Institute for Biological Standards and Control), Schweineinfluenza (A/Swine (HIN2) Bakum/1832/00; IDT Biologika) und Pferdeinfluenza (A/Equine 2 (H3N8); A/Newmarket/1/93; NIBSC, The National Institute for Biological Standards and Control) erfolgte durch Infektion von CAP-1D5 über 4 Passagen in 50 ml Falcon-Behälter in PEM- und 293SFMII-Medium. Vor jeder Infektion wurde ein Mediumwechsel durchgeführt. Die Virusausbeute während den einzelnen Passagen wurde durch Titration des Hämagglutinin (log HA-Einheiten /100 µl) und durch den Tissue Culture Infectious Dose₅₀ Assay (TCID₅₀, Virenzahl/ml) quantifiziert (Genzel and Reichl, Vaccine production - state of the art and future needs in upstream processing in Methods in Biotechnology: Animal Cell Biotechnology - Methods and Protocols : Eds. R. Pörtner; HUMANA Press Inc., Totowa, New Jersey, 2007, 457-473; Kalbfuss et al., 2008; Biologicals 36(3):145-61).

Die Ergebnisse sind in Fig. 5 dargestellt. Die Infektion der CAP-1D5-Zellen mit dem Influenzavirus A/Brisbane/59/2007 und B/Florida/4/2006, führte sowohl im PEM-, als auch im 293SFMII-Medium nach 4 Passagen zu deutlich gesteigerten Virustitern, wobei die Steigerung des Virustiters im 293SFMII-Medium stärker ist. Vergleichbare Zunahme zeigte auch die Titration des HA-Wertes bei Infektion der CAP-1D5- Zellen mit dem Influenzavirus A/Brisbane/59/2007 bzw.

B/Florida/4/2006, sowohl in PEM-, als auch in 293SFMII-Medium. Die Infektion der CAP-ID5-Zellen mit dem Schweineinfluenzavirus führt zu einer Zunahme der Titration des HA-Wertes sowohl in PEM-, als auch in 293SPMII-Medium.

Neben einer Erhöhung des Virustiters über die Adaptation wurde auch mit jeder Passage die Replikation des Virus schneller und es konnte letztlich deutlich der Virustiter gesteigert werden. Generell scheinen in 293SFMII-Medium leicht erhöhte Virustiter im Vergleich zum PEM-Medium erreicht werden.

### Beispiel 7:_Kultivierungsversuche mit der permanenten Amniozytenzelllinie CAP-1D5 bei erhöhter Startzellzahl

Die permanente Amniozytenzelllinie CAP-1D5 wurde in 100 ml PEM-Medium (Invitrogen) bei 37°C, 8% CO₂ und 185 rpm kultiviert. Die Startzellzahl betrug 5 x 10⁵ Zellen/ml bzw. 8 x 10⁵ Zellen/ml. Zusätzlich wurde in den Ansätzen mit einer erhöhten Startzellzahl Pyruvat in einer Endkonzentration von 4 mM bzw. von 10 mM und weitere Aminosäuren hinzugegeben.

Zum Zeitpunkt 0 *(=Startpunkt der Kultur)* sowie jeweils in einer Zeitspanne von 24 h wurde die Lebendzellzahl, die Totzellzahl, die Überlebensrate der Zelllinie, sowie der pH-Wert, die Konzentration an Glucose, Laktose, Glutamin. Ammonium, Glutaminsäure und Pyruvat im Medium (Biochemisches Multiparameter Analysesystem) bestimmt (Lohr et al., Vaccine, 2009, 27(36), 4975-4982; Genzel et al., Appl Microbiol Biotechnol., 2010,88(2):461-75).

Die Ergebnisse sind in Fig. 6 dargestellt. Durch Erhöhung der Startzellzahl von etwa 5 x 10⁵ Zellen pro ml Kultur zu Beginn der Kultivierung auf 8 x 10⁵ Zellen pro ml Kultur der permanenten Amniozytenzelllinie CAP-1D5 in PEM-Medium wurde eine Mehrausbeute von 1 x 10⁶ Zellen pro ml Kultur nach 90 h erzielt. Zum typischen Infektionszeitpunkt (ca. 96 h bis 120 h nach Startpunkt der Kultur) wurden damit eine Zellzahl von 5 - 6 x 10⁶ Zellen pro ml Kultur erreicht.

Der pH-Wert der Kultur lag zum typischen Infektionszeitpunkt (ca, 96 h bis 120 h nach Startpunkt der Kultur) in einem eher kritischen Bereich bei 6,6-6,8. Bevorzugt sollte der pH-Wert bei Infektion bei etwa 7,2-7,4 liegen.

### Beispiel 8. Auswirkungen der Variation der Trypsinaktivität und Durchführung eines Mediumwechsels bzw. einer 1:2-Verdünnung mit Medium auf den Virustiter

In diesem Experiment sollte untersucht werden, wie sich die Verwendung unterschiedlicher Trypsinkonzentrationen bei der Virusinfektion und ein Mediumwechsel oder eine 1:2-Verdünnung des Mediums auf den Virustiter auswirken.

Die permanente Amniozytenzelllinie CAP-1D5 wurde in 100 ml PEM-Medium (Invitrogen) mit jeweils 4 mM Pyruvat und Glutamin bei 37°C, 8% CO₂ und 185 rpm im Schüttelkolben kultiviert. Die Zelllinie wurde zum Startzeitpunkt der Kultur mit an CAP-1D5-Zellen adaptierten A/PR/8/34-Influenzavirus (H1N1 RKI, Robert-Koch-Institut) infiziert. Die Zellzahl in der Kultur betrug zum Zeitpunkt der Infektion 4,5 x 10⁶ Zellen/ml Kultur falls kein Mediumwechsel vor der Infektion stattfand, bzw. 2,3 x 10⁶ Zellen/ml Kultur, falls eine 1:2-Verdünnung mit PEM-Medium vor der Infektion stattfand und 5 x 10⁶ Zellen/ml falls ein kompletter Mediumwechsel vor der Infektion stattfand. Außerdem wurde bei den Kulturen ohne Mediumwechsel und mit einer 1:2-Verdünnung mit PEM-Medium Trypsinkonzentrationen von 1 x 10⁻⁴ U/Zelle, 3 x 10⁻⁵ U/Zelle und 5 x 10⁻⁵ U/Zelle verwendet. Bei den Kulturen mit komplettem Mediumwechsel wurden Trypsinkonzentrationen von 1 x 10⁻⁴ U/Zelle, 1 x 10⁻⁵ U/Zelle, 5 x 10⁻⁵ U/Zelle und 1 x 10⁻⁶ U/Zelle oder kein Trypsin verwendet.

Die Ergebnisse sind in Fig. 7 dargestellt. Die 1:2-Verdünnung mit frischem PEM-Medium führt zu einem früheren Anstieg des HA (ca. 12 h statt 24 h) und zu höheren Maximalwerten des HA- 2,70 log HA verglichen mit 2,30 log HA der Kulturen, bei denen kein Mediumwechsel durchgeführt wurde. Ein kompletter Medienwechsel führte zu erhöhten HA-Werten, die über 3,0 log HA liegen.

In den Kulturen ohne Mediumwechsel und mit einer 1:2-Verdünnung mit PEM-Medium zeigen die log HA-Werte einen sehr ähnlichen Verlauf unabhängig von der Trypsinkonzentration. In den Kulturen, in denen ein kompletter Mediumwechsel durchgeführt wurde, ist bei den Trypsinkonzentrationen 1 x 10⁻⁵ U/Zelle, 5 x 10⁻⁵ U/Zelle und 1 x 10⁻⁶ U/Zelle der Verlauf der log HA-Werte sehr äbnlich. In der Kultur ohne Trypsin erreichte der log HA-Wert lediglich einen Wert von etwa 2 log HA-Einheiten/100µl und in der Kultur, in der 1 x 10⁻⁴ U/Zelle Trypsin für die Infektion verwendet wurde, lag der log HA-Wert unter 1 log HA-Einheit/100µl.

### Beispiel 9: MOI-(multiplicily of infection) Abhängigkeit der Infektion von CAP-1D5-Zellen in PEM-Medium mit unterschiedlichen adaptierten Influenzavirusstämmen mit und ohne Durchführung eines Mediumwechsels vor Infektion

Mit dem vorliegenden Experiment wurde die Abhängigkeit zwischen dem MOI-Wert, d.h. dem zahlenmäßigen Verhältnis aus der zur Infektion verwendeten Viruspartikelanzahl und der Anzahl der zu infizierenden CAP-1D5-Zellen und dem Virustiter (in log HA-Einheiten pro 100 µl Kultur) untersucht.

Hierfür wurde die permanente Amniozytenzelllinie CAP-1D5 in 50 ml PEM-Medium (Invitrogen) mit jeweils 4 mM Pyruvat und Glutamin bei 37°C, 8% CO₂ und 185 rpm im Schüttelkolben kultiviert. Die MOI-Abhängigkeit wurde sowohl ohne Mediumwechsel und mit Mediumwechsel der Kultur vor der Infektion überprüft. Es wurden drei verschiedene adaptierte Influenzastämme verwendet: A/PR/8/34 (RKI, Robert-Koch-Institut). A/Brisbane/59/2007 (IVR-148, NIBSC, The National Institute for Biological Standards and Control) und B/Florida/4/2006 (NIBSC. The National Institute for Biological Standards and Control). Die Infektion erfolgte in 50 mL Schüttelkolben mit einer Zellkonzentration bei Beimpfung von 4,9 x 10⁶ Zellen/mL (ohne Mediumwechsel) und 5,0 x 10⁶ Zellen/mL (mit Mediumwechsel). Die Infektion erfolgte bei MOI-Werten von 0,25 bzw. 0,10 (bei A/Brisbane-Influenzavirus). 0.025 und 0,0025, wenn kein Mediumwechsel durchgeführt wurde und bei MOI-Werten von 0.10 bzw. 0,06 (bei A/Brisbane-Influenzavirus), 0,025 und 0,0025, falls ein Mediumwechsel durchgeführt wurde. Die Trypsinaktivität betrug ohne Mediumwechsel 1 x 10⁻⁴ U/Zelle und mit Mediumwechsel 1 x 10⁻⁶ U/Zelle. Anschließend wurde über 144 h hinweg die Menge der Viruspartikel (log HA-Einheiten/100 µl) bestimmt (Kalbfuss et al., 2008; Biologicals 36(3):145-61).

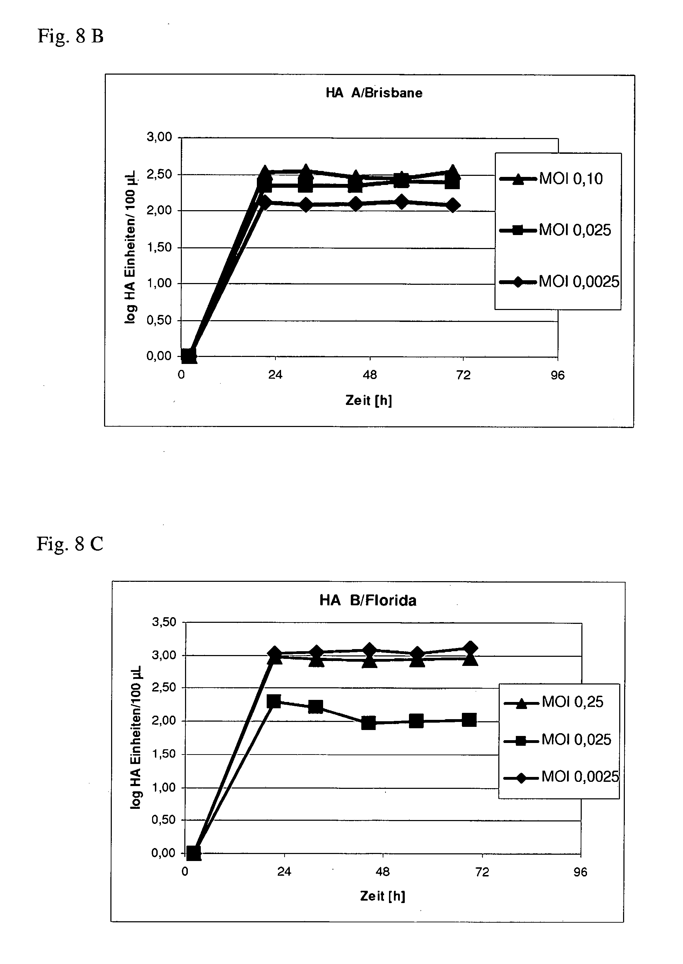

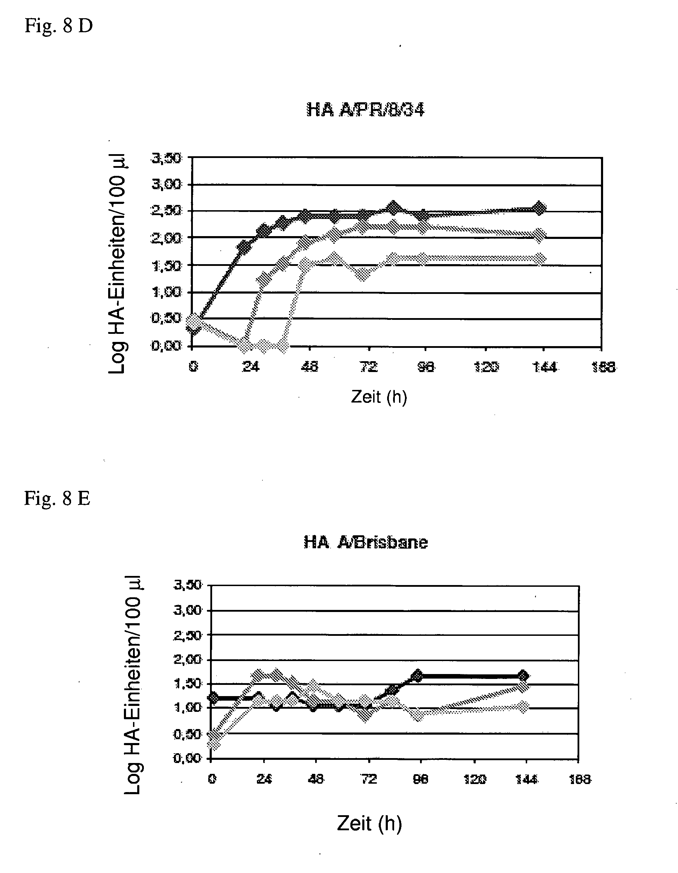

Die Ergebnisse sind in Fig. 8 dargestellt. Die Durchführung eines Mediumwechsels führt zu konsistenteren Ergebnissen bei der Virusreplikation. Eine gerine MOI-Abhängigkeit ist nur bei den mit influenzavirus A/PR/8/34 infizierten Zellen ohne Mediumwechsel und bei den mit Influenzavirus A/Brisbane infizierten Zellen mit Mediumwechsel zu erkennen. Mit Mediumwechsel können deutlich höhere log HA-Werte erreicht werden. Die pH-Werte liegen ohne Mediumwechsel teilweise im kritischen Bereich zwischen 6.6 und 6,8, und bei Mediumwechsel bei 7,3-7,5.

Beispiel 10: Weiterführende Kultivierung im 1 L Maßstab in STR- und Wave-Bioreaktoren mit Infektion (A/PR/8/34 adaptiert)

In einem Ansatz (B16) wurden CAP-1D5-Zellen im 1 Liter Bioreaktor STR (Sartorius) in PEM-Medium mit 4 mM Glutamin und 4 mM Pyruvat bei 120 rpm, pH = 7,2 und einem Sauerstoffpartialdruck pO₂ von 40% mit reinem Sauerstoff kultiviert. Die Startzellzahl betrug 5 x 10⁵ Zellen/mL. Nach 72,75 h Wachstum und einer Zellzahl von 2,1 x 10⁶ Zellen/ml wurden die CAP-1D5-Zellen mit Influenzavirus A/PR/8/34 (adaptiert: in PEM, 4. Passage, 2.01 x 10⁶ Viren/mL) infiziert. Dabei wurde kein Mediumwechsel vorgenommen. Der MOI-Wert betrug 0,025 und es wurde Trypsin in einer Endkonzentration von 3 x 10⁻⁵ U/mL zugegeben.

In einem weiteren Ansatz (B26) wurden CAP-1D5-Zellen im 1 Liter Bioreaktor STR (Sartorius) in PEM-Medium bei 120 rpm, pH = 7,4-7,2 und einem Sauerstoffpartialdruck pO₂ von 40% mit reinem Sauerstoff kultiviert. Die Startzellzahl betrug 8 x 10⁵ Zellen/mL. Nach 92 h Wachstum wurden die CAP-1D5-Zellen mit Influenzavirus A/PR/8/34 (adaptiert: in PEM, 4. Passage, 3,75 x 10⁶ Viren/mL) infiziert. Zuvor wurde ein kompletter Mediumwechsel vorgenommen und der pH-Wert auf 7,6 eingestellt. Der MOI-Wert betrug 0,025 und es wurde Trypsin in einer Endkonzentration von 3 x 10⁻⁵ U/mL zugegeben.

In einem dritten Ansatz (wave) wurden CAP-1D5-Zellen im 1 Liter Wave Bioreaktor (Wave Biotech AG) in PEM-Medium mit 4 mM Glutamin, 4 mM Pyruvat und 20 mM Glucose bei einer Wippfrequenz von 13 rpm, bei einem Winkel von 7°, pH = 7,3-6,9 und einem Sauerstoffpartialdruck pO₂ von 40% mit reinem Sauerstoff und einem CO₂-Partialdruck von 7,5 % kultiviert. Die Startzellzahl betrug 5 x 10⁵ Zellen/mL. Nach 72 h Wachstum wurden die CAP-1D5-Zellen mit Influenzavirus A/PR/8/34 (adaptiert: in PEM, 4. Passage, 1,87 x 10⁶ Zellen/ml) infiziert. Die Zellzahl vor Infektion betrug 2,1 x 10⁶ Zellen/ml. Dabei wurde kein Mediumwechsel vorgenommen. Der MOI-Wert betrug 0,025 und es wurde Trypsin in einer Endkonzentration von 3 x 10⁻⁵ U/mL zugegeben.

In einem vierten Ansatz wurden MDCK.SUS2- Zellen im 1 Liter Bioreaktor STR (Sartorius) in AEM-Medium kultiviert. Die Startzellzahl betrug 5 x 10⁵ Zellen/mL. Nach 118,25 h Wachstum wurden die MDCK.SUS2-Zellen mit Influenzavirus A/PR/8/34 infiziert (Lohr et al., Vaccine, 2010, 28(38):6256-64).

Die Ergebnisse sind in Fig. 9 dargestellt. Über 192 h hinweg wurde die Lebendzellzahl, und die Totzellzahl bestimmt, sowie der pH-Wert, die Konzentration an Glucose. Laktose, Glutamin, Ammonium, Glutaminsäure und Pyruvat im Medium. Weiter wurden ab dem Zeitpunkt der Infektion die log HA-Einheiten/100 µl und TCID₅₀-Werte ermittelt (Genzel and Reichl, Vaccine production - state of the art and future needs in upstream processing in Methods in Biotechnology: Animal Cell Biotechnology - Methods and Protocols; Eds. R. Pörtner, HUMANA Press Inc., Totowa, New Jersey, 2007, 457-473; Kalbfuss et al., 2008; Biologicals 36(3):145-61).

Die CAP-1D5-Zellen wachsen in allen drei Ansätzen schneller und in höherer Dichte verglichen mit den MDCK.SUS2-Zellen. Die Virustiter in den CAP-1D5-Zellkulturen erreichen maximal einen Wert für die log HA-Einheiten/100 µl von etwa 2,5. Der Virustiter der MDCK.SUS2-Zellkultur erreicht einen maximalen Wert für die log HA-Einheiten/100 µl von etwa 3. Die Virustiter in den CAP-1D5-Zellkulturen steigen verglichen mit dem Virustiter in der MDCK.SUS2-Zellkultur deutlich früher an.

### Beispiel 11: Kultivierungsversuch zur Steigerung der Virusausbeute im Schüttelkolben in PEM- oder 293SFMII-Medium mit komplettem oder 1:1-Mediumwechsel vor Infektion

Zur Optimierung der Virusausbeute in CAP-1D5-Zellkulturen, die in Schüttelkolben kultiviert werden, wurden die CAP-1D5-Zellen in den Medien 293SFMII (Invitrogen) und PEM (Invitrogen) kultiviert und ein 1:1-Medienwechsel oder ein kompletter Medieawechsel durchgeführt.

Die permanente Amniozytenzelllinie CAP-1D5 wurde in 50 ml PEM-Medium mit 4 mM Glutamin und 4 mM Pyruvat bei 37°C, 8% CO₂ und 100 rpm im 100 ml Scbüttelkolben kultiviert Vor Infektion der Zellen mit adaptiertem Influenzavirus A/PR/8/34 bei einer MOI von 0.025 wurde ein Mediumwechsel durchgeführt. Wird ein 1:1-Mediumwechsel durchgeführt, wird bei Infektion der Zellen Trypsin in einer Konzentration von 1 x 10⁻⁵ U/Zelle verwendet. Wird ein kompletter Mediumwechsel durchgeführt, wird bei Infektion der Zellen Trypsin in einer Konzentration von 1 x 10⁻⁶ U/Zelle verwendet. Der Mediumwechsel erfolgte sowohl mit PEM-Medium, wie auch mit 293SFMII-Medium. Die Zellkonzentration bei Infektion betrug 5 x 10⁶ Zellen/ml.

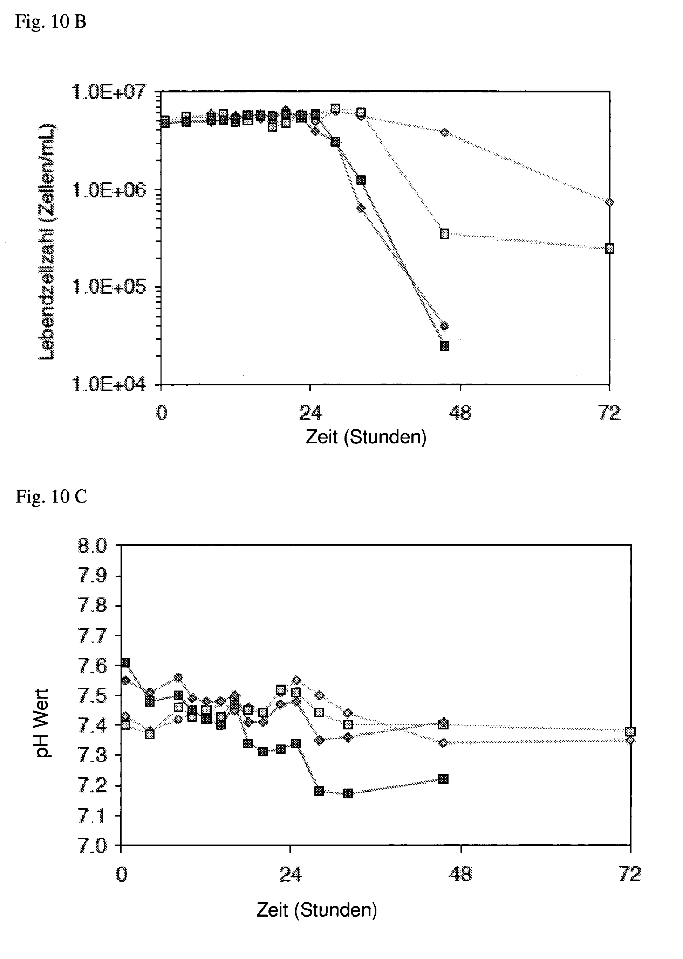

Die Ergebnisse sind in Fig. 10 dargestellt. Ober 72 h hinweg, wurde die Lebendzellzahl, die Überlebensrate, der pH-Wert, sowie die log HA-Einheiten/100 µl durch Titration des Hämagglutinins mittels Standardverfahren ermittelt (Lohr et al., Vaccine, 2009, 27(36), 4975-4982; Genzel et al., Appl Microbiol Biotechnol., 2010 ,88(2):461-75).

Der Virustiter stieg in den Zellkulturen, in denen vor der Infektion mit Influenzavirus A/PR/8/34 ein kompletter Mediumwechsel durchgeführt wurde, schneller an im Vergleich zu den Zellkulturen, in denen ein 1:1-Mediumwechsel erfolgte. Außerdem erreichten die Virustiter der Zellkulturen, in denen vor der Infektion mit Influenzavirus A/PR/8/34 ein kompletter Mediumwechsel durchgeführt wurde, einen höheren maximalen Virustiter verglichen mit den Zellkulturen, in denen ein 1:1-Mediumwechsel erfolgte.

Die Lebendzellzahl der Zellkulturen mit komplettem Mediumwechsel vor Infektion fiel von 5 x 10⁶ Zellen/ml nach 24 h steil ab, so dass sie nach 48 h etwa noch 2 x 10⁴ Zellen/ml betrug. Am wenigsten stark fiel die Zellkultur mit PEM-Medium und 1:1 Mediumwechsel vor Infektion, hierbei betrug die Lebendzellzahl nach 72 h etwa noch 7 x 10⁵ Zellen/ml.

Der pH-Wert lag in allen Zellkulturen über die gesamte Zeit von 72 h zwischen 7,6 und 7,2.

## Patentansprüche

1. Verfahren zur Herstellung eines Influenzavirus-basierten Impfstoffs umfassend
a) Infizieren einer permanenten humanen Amniozytenzelle mit einem Influenzavirus,
b) Züchten der permanenten humanen Amniozytenzelle,
c) Expression des Influenzavirus und
d) Isolieren des Influenzavirus aus dem Medium,
wobei die permanente humane Amniozytenzelle die adenoviralen Genprodukte E1A und E1B exprimiert.

2. Verfahren zur Herstellung eines Influenzavirus-basierten Impfstoffs nach Anspruch 1, wobei sich die permanente humane Amniozytenzelle zum Zeitpunkt des Infizierens mit dem Influenzavirus in oder zwischen der exponentiellen Wachstumsphase und der stationären Wachstumsphase befindet.

3. Verfahren zur Herstellung eines Influenzavirus-basierten Impfstoffs nach Anspruch 1, wobei das Isolieren des Influenzavirus aus dem Medium unter Schritt d) mittels Dichtegradienten-Differential- oder Zonenzentrifugation erfolgt.

4. Verfahren zur Herstellung eines Influenzavirus-basierten Impfstoff nach Anspruch 1, wobei die adenoviralen Genprodukte E1A und E1B die Nukleotide 1 bis 4344, 505 bis 3522 oder die Nukleotide 505 bis 4079 des humanen Adenovirus Serotyp-5 umfassen.

5. Verfahren zur Herstellung eines Influenzavirus-basierten Impfstoffs nach einem der vorhergehenden Ansprüche, wobei die permanente humane Amniozytenzelle das adenovirale Genprodukt pIX exprimiert.

6. Verfahren zur Herstellung eines Influenzavirus-basierten Impfstoffs nach einem der vorhergehenden Ansprüche, wobei vor dem Infizieren mit einem Influenzavirus ein kompletter Mediumwechsel oder eine 1:2-Verdünnung mit Medium stattfindet.

7. Verfahren zur Herstellung eines Influenzavirus-basierten Impfstoffs nach einem der vorhergehenden Ansprüche, wobei beim Infizieren mit einem Influenzavirus eine Trypsinkonzentration von 1 x 10⁻⁴ U/Zelle, 1 x 10⁻⁵ U/Zelle, 3 x 10⁻⁵ U/Zelle, 5 x 10⁻⁵ U/Zelle oder 1 x 10⁻⁶ U/Zelle zugegeben wird.

8. Verfahren zur Herstellung eines Influenzavirus-basierten Impfstoffs nach einem der vorhergehenden Ansprüche, wobei beim Infizieren mit einem Influenzavirus, eine Virusmenge angegeben als MOI-Wert im Bereich von 0,001 bis 0,3 verwendet wird.

9. Verfahren zur Herstellung eines Influenzavirus-basierten Impfstoffs nach einem der vorhergehenden Ansprüche, wobei das Influenzavirus ein humanes Influenzavirus, ein Pferdeinfluenzavirus oder ein Schweineinfluenzavirus ist.

10. Verfahren zur Herstellung eines Influenzavirus-basierten Impfstoffs nach einem der vorhergehenden Ansprüche, wobei das Influenzavirus ausgewählt ist aus den Influenzavirusstämmen A/PR/8/34, A/Uruguay/716/2007, A/Brisbane/59/2007, B/Florida/4/2006, Schweineinfluenza (A/Swine (H1N2) Bakum/1832/00) oder Pferdeinfluenza (A/Equine, A/Newmarket/1/93 (H3N8)).

11. Verwendung einer permanenten humanen Amniozytenzelle zur Herstellung eines Influenzavirus-basierten Impfstoffs, wobei die permanente humane Amniozytenzelle die adenoviralen Genprodukte E1A und E1B exprimiert.

## Claims

1. A method for the production of an influenza virus based vaccine, comprising
a) infecting a permanent human amniocyte cell with an influenza virus;
b) culturing the permanent human amniocyte cell;
c) expression of the influenza virus; and
d) isolating the influenza virus from the medium,
wherein the permanent human amniocyte cell expresses the adenoviral gene products E1A and E1B.

2. The method for the production of an influenza virus based vaccine according to claim 1, wherein the permanent human amniocyte cell is in or between the exponential growth phase and the stationary growth phase at the time of infecting with the influenza virus.

3. The method for the production of an influenza virus based vaccine according to claim 1, wherein the isolation of the influenza virus from the medium in step d) takes place by means of density gradient differential or zonal centrifugation.

4. The method for the production of an influenza virus based vaccine according to claim 1, wherein the adenoviral gene products E1A and E1B comprise the nucleotides 1 to 4344, 505 to 3522 or the nucleotides 505 to 4079 of the human adenovirus serotype-5.

5. The method for the production of an influenza virus based vaccine according to any of the previous claims, wherein the permanent human amniocyte cell expresses the adenoviral gene product pIX.

6. The method for the production of an influenza virus based vaccine according to any of the previous claims, wherein a complete medium change or a 1:2 dilution with medium takes place prior to the infection with an influenza virus.

7. The method for the production of an influenza virus based vaccine according to any of the previous claims, wherein a trypsin concentration of 1 x 10⁻⁴ U/cell, 1 x 10⁻⁵ U/cell, 3 x 10⁻⁵ U/cell, 5 x 10⁻⁵ U/cell or 1 x 10⁻⁶ U/cell is added when infecting with an influenza virus.

8. The method for the production of an influenza virus based vaccine according to any of the previous claims, wherein a virus amount indicated as MOI value in the range of 0.001 to 0.3 is used, when infecting with an influenza virus.

9. The method for the production of an influenza virus based vaccine according to any of the previous claims, wherein the influenza virus is a human influenza virus, an equine influenza virus or a swine influenza virus.

10. The method for the production of an influenza virus based vaccine according to any of the previous claims, wherein the influenza virus is selected from the influenza virus strains A/PR/8/34, A/Uruguay/716/2007, A/Brisbane/59/2007, B/Florida/4/2006, swine influenza (A/Swine (H1N2) Bakum/1832/00) or equine influenza (A/Equine, A/Newmarket/1/93 (H3N8)).

11. Use of a permanent human amniocyte cell for the production of an influenza virus based vaccine, wherein the permanent human amniocyte cell expresses the adenoviral gene products E1A and E1B.

## Revendications

1. Procédé de production d'un vaccin à base de virus de la grippe comprenant
a) l'infection d'un amniocyte humain permanent par un virus de la grippe,
b) la culture de l'amniocyte humain permanent,
c) l'expression du virus de la grippe et
d) l'isolement du virus de la grippe à partir du milieu,
dans lequel l'amniocyte humain permanent exprime les produits géniques adénoviraux E1A et E1B.

2. Procédé de production d'un vaccin à base de virus de la grippe selon la revendication 1, dans lequel l'amniocyte humain permanent se trouve en phase de croissance exponentielle ou entre la phase de croissance exponentielle et la phase de croissance stationnaire au moment de l'infection par le virus de la grippe.

3. Procédé de production d'un vaccin à base de virus de la grippe selon la revendication 1, dans lequel l'isolement du virus de la grippe à partir du milieu se fait à l'étape d) au moyen d'une centrifugation différentielle ou de zone en gradients de densité.

4. Procédé de production d'un vaccin à base de virus de la grippe selon la revendication 1, dans lequel les produits géniques adénoviraux E1A et E1B comprennent les nucléotides 1 à 4344, 505 à 3522 ou les nucléotides 505 à 4079 de l'adénovirus humain sérotype 5.

5. Procédé de production d'un vaccin à base de virus de la grippe selon une des revendications précédentes, dans lequel l'amniocyte humain permanent exprime le produit génique adénoviral pIX.

6. Procédé de production d'un vaccin à base de virus de la grippe selon une des revendications précédentes, dans lequel un changement de milieu complet ou une dilution à 1/2 avec le milieu s'effectue avant l'infection par le virus de la grippe.

7. Procédé de production d'un vaccin à base de virus de la grippe selon une des revendications précédentes, dans lequel une concentration en trypsine de 1 x 10⁻⁶ U/cellule, 1 x 10⁻⁵ U/cellule, 3 x 10⁻⁵ U/ cellule, 5 x 10⁻⁵ U/cellule ou 1 x 10⁻⁶ U/cellule est ajoutée lors de l'infection par le virus de la grippe.

8. Procédé de production d'un vaccin à base de virus de la grippe selon une des revendications précédentes, dans lequel une quantité de virus indiquée sous forme de valeur MOI dans la plage de 0,001 à 0,3 est utilisée lors de l'infection par un virus de la grippe.

9. Procédé de production d'un vaccin à base de virus de la grippe selon une des revendications précédentes, dans lequel le virus de la grippe est un virus de la grippe humain, un virus de la grippe équine ou un virus de la grippe porcine.

10. Procédé de production d'un vaccin à base de virus de la grippe selon une des revendications précédentes, dans lequel le virus de la grippe est choisi parmi les souches de virus de la grippe A/PR/8/34, A/Urugay/716/2007, A/Brisbane/59/2007, B/Florida/4/2006, grippe porcine (A/Swine (H1N2) Bakum/1832/00) ou grippe équine (A/Equine, A/Newmarket/1/93 (H3N8)).

11. Utilisation d'un amniocyte humain permanent pour la production d'un vaccin à base de virus de la grippe, dans laquelle l'amniocyte humain permanent exprime les produits géniques adénoviraux E1A et E1B.
